(19) Europäisches Patentamt
European Patent Office
Office européen des brevets

(11) Publication number: **0 146 348 B1**

(12) **EUROPEAN PATENT SPECIFICATION**

(45) Date of publication of patent specification: **31.07.91**

(51) Int. Cl.$^5$: **C07C 229/34**, C07C 43/23, C07C 69/712, C07D 209/08, C07D 307/79, C07D 333/54, A61K 31/215, A61K 31/075, A61K 31/40, A61K 31/34

(21) Application number: **84308683.6**

(22) Date of filing: **13.12.84**

(54) **Substituted naphthalenes, indoles, benzofurans and benzothiophenes as lipoxygenase inhibitors.**

(30) Priority: **14.12.83 US 561601**
**05.11.84 US 668111**

(43) Date of publication of application:
**26.06.85 Bulletin 85/26**

(45) Publication of the grant of the patent:
**31.07.91 Bulletin 91/31**

(84) Designated Contracting States:
**BE CH DE FR GB IT LI NL SE**

(56) References cited:
**EP-A- 0 039 880**

**EUROPEAN JOURNAL OF MEDICINAL CHEMISTRY, Chimica Therapeutica, vol. 16, no. 6, November/December 1981, pages 563-568; G. BASTIAN et al.: "Recherches sur les dérivés nitrés d'intérêt biologique. XXIV. Synthèse et étude photobiologique de turocoumarines nitrées"**

(73) Proprietor: **THE UPJOHN COMPANY**
**301 Henrietta Street**
**Kalamazoo, Michigan 49001(US)**

(72) Inventor: **Yamashita, Ayako c/o The Upjohn Company**
**301 Henrietta Street**
**Kalamazoo, Michigan 49001(US)**

(74) Representative: **Perry, Robert Edward et al**
**GILL JENNINGS & EVERY 53-64 Chancery Lane**
**London WC2A 1HN(GB)**

**EP 0 146 348 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to substituted naphthalenes, indoles, benzofurans and benzothiophenes (many of which are novel) which are useful as inhibitors of the synthesis of leukotrienes and as inhibitors of the action of lipoxygenase in mammalian metabolism.

The leukotrienes are a class of unsaturated fatty acid compounds which are derived from arachidonic acid by the action of lipoxygenase. Some are potent constrictors of human bronchi, mediating the action of slow-reacting substance of anaphylaxis (SRS-A); see Dahlen, Nature 288 (1980) 484.

Leukotrienes may act as agonists in immediate hypersensitivity and other pathological conditions. Leukotriene $C_4$ ($LTC_4$) and leukotriene $D_4$ ($LTD_4$), especially, are potent mucous secretagogues. Shelhamer et al., Chest 81 (1982) 36S, summarise the nature of evidence available suggesting that lipoxygenase products generated by the airways in vitro might be responsible for the augmented mucus release.

In mammalian metabolism, arachidonic acid is transformed to 12-L-hydroperoxy-5,8,10,14-eicosatetraenoic acid by the action of 12-lipoxygenase. Similarly, 5-lipoxygenase transforms arachidonic acid into 5-S-hydroperoxy-6,8,11,14-eicosatetraenoic acid. Thus, an agent which inhibits the action of lipoxygenase may be useful in treating or preventing untoward conditions associated with lipoxygenase products.

A number of substituted 1-hydroxy-4-methoxy-naphthalenes (see formula I: $R_3$ = H) are known. Thus, K.H. Dotz et al, J. Organometal. Chem. 247(2):187-201 (1983), discloses 4-methoxy-1-naphthalenols substituted in the 2- and 3-positions by phenyl, substituted phenyl, methyl, ethyl, octyl and propyl. 4-Methoxy-2-phenyl-1-naphthalenol is disclosed by K. Buggle et al, J. Chem. Soc., Perkin-Trans 1 (6):572-575 (1975). 4-Methoxy-2,3-diphenyl-1-naphthalenol is disclosed by K.H. Dotz, J. Organmet. Chem. 140(2):177-86 (1977). 4-Methoxy-2,3-dimethyl-1-naphthalenol is disclosed by K.H. Dotz et al, Chem. Ber. 110(4):1555-63 (1977). 2-Tert-butyl-4-methoxy-1-naphthol is disclosed in Japanese Patent 7010339. US-A-3948958 discloses 4-methoxy-3-methyl-1-naphthenol. 4-Methoxy-2-methyl-1-naphthenol is disclosed by I.D. Snyder et al, J. Am. Chem. Soc. 96(26):8046-53 (1974). The 2,3-unsubstituted 4-methoxy-1-naphthenol compound is also known; see GB-A-1122085.

European Journal of Medicinal Chemistry, Chimica Therapeutica 16(6):563-568 (November/December 1981), discloses as its compound 39 the compound of formula I wherein $R_1$, $R_2$ and $R_3$ are all H and D is -O-.

Certain 1-acetoxy-4-methoxy-naphthalenes (I: $R_3$ = $OCCH_3$) are also known. Thus, 4-methoxy-1-naphthalenol acetate is disclosed in DE-A-2802666. The corresponding 2,3-dimethyl compound is disclosed by F.M. Dean et al, J. Chem. Soc., Perkin Trans. 1(20):2289-94 (1977). 4-Methoxy-2-phenyl-1-naphthalenol is disclosed by O. Gonclalves de Lima et al, Rev. Inst. Antibiot., Univ. Recife 5(1-2):3-9 (1963).

EP-A-0039880 discloses compounds of formula Ia wherein $R_1$ is H or alkyl, $R_2$ is H and D is -CH=CH- as in vitro diagnostics.

According to a first aspect of the present invention, compounds for therapeutic use are of formula Ia, wherein $R_1$ and $R_2$ are independently selected from H, $C_{1-10}$ alkyl, $C_{2-10}$ alkenyl and PhX which represents phenyl optionally substituted by up to 3 substitutents independently selected from $C_{1-4}$ alkyl, Cl, F, Br, $NO_2$, $CF_3$, OH and $C_{1-4}$ alkoxy;

D is -CH=CH-, -N($CH_3$)-, -S- or -O-; and

AA is part of an amino-acid of the formula HOOC-AA;

with the proviso that $R_1$ and $R_2$ are not both phenyl when D is -N($CH_3$)-;

and include pharmacologically acceptable acid addition salts thereof.

According to a second aspect of the present invention, compounds for therapeutic use are of formula I, wherein $R_1$, $R_2$ and D are as defined above; and

$R_3$ is H or $OCCH_3$;

with the proviso that $R_3$ is not H when D is -N($CH_3$)-, and that $R_1$ and $R_2$ are not both phenyl when D is -N($CH_3$)-.

According to a third aspect of the present invention, novel compounds of formula I are as defined above, with the additional provisos that $R_3$ is not H when D is -CH=CH-, that $R_1$ is neither H nor $CH_3$ when $R_2$ is H or $CH_3$ and D is -CH=CH-, that $R_1$ is not H when $R_2$ is phenyl and D is -CH=CH-, and that $R_1$, $R_2$ and $R_3$ are not each H when D is -O-.

According to a fourth aspect of the present invention, novel compounds are of formula Ia with the additional proviso that $R_1$ is not H or alkyl when $R_2$ is H and D is -CH=CH-.

Preferred compounds of formula Ia are those wherein D is -CH=CH- and AA is $-(CR_{17}R_{18})_m-(CH_2)_n-NR_{14}R_{15}$, PhX-$NH_2$ or a naturally-occurring amino-acid minus its carboxylic acid function, in which m is 1, 2, 3 or 4, n is 0, 1, 2, 3, 4 or 5, $R_{14}$ and $R_{15}$ are independently selected from H, $C_{1-10}$ alkyl, ($C_{1-4}$ alkyl)-

carbonyl, -CO-PhX and -PhX, the or each $R_{17}$ and the or each $R_{18}$ are independently selected from H, $C_{1-10}$ alkyl, $-CH_2$-PhX and -PhX, and PhX-$NH_2$ represents PhX ring-substituted by $NH_2$, PhX being as defined above.

It is generally preferred that D is -CH=CH-. Another preference is that $R_1$ and $R_2$ are each ethyl or $R_1$ is H and $R_2$ is n-butyl.

The carbon atom content of various hydrocarbon-containing moieties is indicated by a prefix designating the minimum and maximum number of carbon atoms in the moiety, i.e., the prefix ($C_i$-$C_j$) indicates a moiety of the integer "i" to the integer "j" carbon atoms, inclusive. Thus ($C_1$-$C_3$)alkyl refers to alkyl of one to 3 carbon atoms, inclusive, or methyl, ethyl, propyl, and isopropyl.

Examples of alkyl of one to 10 carbon atoms, inclusive, are methyl, ethyl, propyl, butyl, pentyl, hexyl, heptyl, octyl, nonyl, decyl, and isomeric forms thereof.

Examples of $C_2$-$C_{10}$ alkenyl are allyl, 1-methylallyl, 2-methylallyl (methallyl), 2-butenyl (crotyl), 3-butenyl, 1,2-dimethylallyl, 1,1-dimethylallyl, 2-ethylallyl, 1-methyl-2-butenyl, 2-methyl-2-butenyl, 3-methyl-2-butenyl, 3-pentenyl, 2,3-dimethyl-2-butenyl, 1,1,2-trimethylallyl, 1,3-dimethyl-2-butenyl, 1-ethyl-2-butenyl, 4-methyl-2-pentenyl, 2-ethyl-2-pentenyl, 4,4-dimethyl-2-pentenyl, 2-heptenyl, 2-octenyl, 5-octenyl, 1,4-dimethyl-1-hexenyl, and the like.

Examples of Phx are phenyl, p-chlorophenyl, m-bromophenyl, 2,4-difluorophenyl, 2,4,6-trichlorophenyl, p-tolyl, m-tolyl, o-tolyl,p-ethylphenyl, p-tert-butylphenyl, 2,5-dimethylphenyl, 4-chloro-2-methylphenyl, 2,4-dichloro-3-methylphenyl, p-nitrophenyl, p-methoxyphenyl, 3-trifluorophenyl, and 4-hydroxyphenyl.

Examples of acids, which are commonly used for salt formation, are hydrochloric acid, hydrobromic acid, hydroiodic acid, methanesulfonic acid, ethanesulfonic acid, benzenesulfonic acid, phosphoric acid, acetic acid, propionate acid, succinic acid, para-toluenesulfonic acid, maleic acid, tartaric acid, and lactic acid.

By -C(O)-AA is meant the acyl part of an amino acid including the naturally-occurring acids such as: glycine, alanine, valine, leucine, isoleucine, phenylalanine, lysine, proline, tryptophan, methionine, serine, threonine, cysteine, tyrosine, asparagine, glutamine, aspartic acid, glutamic acid, arginine, ornithine, and histidine, and synthetic derivatives thereof. These compounds may be in L or D configuration and are well known and readily available to those skilled in the art. Thus, AA-COOH would represent the amino acids themselves.

In formula Ia, the AA substituent is an amino acid derivative which may be in the "D" and/or "L" configuration, the prefixes "D" and "L" are a means of indicating the relative configurations of various optically active compounds, especially carbohydrates. The compound glyceraldehyde, $CH_2OHCHOHCHO$, was selected as a standard of reference, because it is the simplest carbohydrate - an aldotriose - capable of optical isomerism. (+)-Glyceraldehyde was arbitrarily assigned a configuration and was designated D-glyceraldehyde, and (-)-glyceraldehyde was assigned a second configuration and was designated L-glyceraldehyde. R. T. Morrison & R. N. Boyd, Organic Chemistry 1087-88 (1978). Compounds related configurationally to D-glyceraldehyde are given the designation D, and compounds related to L-glyceraldehyde are given the designation L. Organic Chemistry at 1089. In the present invention, both L and D configurations are observed; however, the L-configuration predominates and is preferred.

The compounds of the present invention will be named herein using the Chemical Abstracts numbering system (see Naming and Indexing of Chemical Substances for Chemical Abstracts during the Ninth Collective Period (1972-1976), a reprint of section IV from the Volume 76 Index Guide.) When D is -CH=CH-, the compounds are named as naphthalenes. When D is $=N(CH_3)$, the compounds are named as N-methyl indoles, and when D is -O- and -S-, the compounds are named as benzofurans and benzothiophenes, respectively.

As noted, compounds of this invention are useful to inhibit the formation of slow reacting substance - anaphylaxis (SRS-A) and thus its smooth muscle contracting and secretory effects.

To demonstrate the SRS-A inhibitory activity of the compounds of this invention, compounds of this invention were evaluated in a standard laboratory test. This test is conducted in rat mononuclear cells incubated in the presence of cysteine and challenged with a calcium ionophore (which induces SRS-A formation). Among the non-amino compounds of this invention, in this test system, 1-acetoxy-2-n-butyl-4-methoxynaphthalene (Example 3) and 1-acetoxy-2,3-diethyl-4-methoxynaphthalene (Example 4) are preferred. At a concentration of 10 micrograms/ml, these compounds inhibited the synthesis of SRS-A 100% and 95%, respectively.

Certain of the amino acyl compounds of this invention (formula Ia) were evaluated in a standard laboratory test to measure the inhibition of leukotriene synthesis. Four of the amino-type compounds of this invention were preferred in this test. Specifically, in this test system, L-Valine, 2,3-diethyl-4-methoxynaphth-1-yl ester, hydrochloride; L-Valine, 2-n-butyl-4-methoxynaphth-1-yl ester; L-Valine, 2-n-butyl-4-methoxynaphth-1-yl ester, hydrochloride; and L-Alanine, 2-n-butyl-4-methoxynaphth-1-yl ester were preferred. At a concentration of 1 microgram/ml, these compounds inhibited the synthesis of leukotriene 100%, 97%, 93%, and 98%, respectively. At a concentration of 10 micrograms/ml, these compounds completely inhibited the synthesis of leukotriene.

The amino acyl compounds of this invention are also preferred for drug formulation because they are more easily crystallizable (particularly, the salts) and are more water soluble. On the basis of biological activity and ease of formulation, L-Valine, 2,3-diethyl-4-methoxynaphth-1-yl ester, hydrochloride is the most preferred.

The compounds of the present invention were also tested for lipoxygenase inhibition. Arachidonic acid is added to washed human platelets and the oxygen uptake is measured using oxygraph cells. A decrease of oxygen uptake versus the control cell indicates inhibition of lipoxygenase. For a full description of the procedure see, Wallach, et al., Biochim. Biophys. Acta. 231:445 (1976).

Using this system, 1-acetoxy-2-n-butyl-4-methoxynaphthalene (Example 3) and 1-acetoxy-2,3-diethyl-4-methoxynaphthalene (Example 4) are preferred, both compounds completely inhibiting 5-HETE production at 10 $\mu$M. The former compound exhibits an $IC_{50}$ of approximately $10^{-7}$M.

Thus, some of the novel compounds of this invention have been shown to be active as inhibitors of the production of leukotrienes and some of the compounds of this invention have been shown to be active as inhibitors of the lipoxygenase enzyme system. Some of these compounds are effective in both systems. All of the compounds of this invention are active as inhibitors of at least one of these two systems. Accordingly, these novel compounds are useful for administration to mammals, including humans, whenever it is desirable medically to inhibit either of these systems. Inhibitors of either system are useful in the treatment of asthma.

Thus, all of the compounds of this invention are useful in the treatment of asthma. For example, these compounds are useful as broncho-dilators or as inhibitors of mediators such as SRS-A which are released from cells activated by an antigen-antibody complex. Thus, these compounds control spasm and facilitate breathing in conditions such as bronchial asthma, bronchitis, bronchiectasis, pneumonia and emphysema. For these purposes, these compounds are administered in a variety of dosage forms, e.g., orally in the form of tablets, capsules, or liquids; rectally in the form of suppositories; parenterally, subcutaneously, or intramuscularly, with intravenous administration being preferred in emergency situations, by inhalation in the form of aerosols or solutions for nebulizers; or by insufflation in the form of powder. Doses in the range of about 0.01 to 50 mg per kg of body weight are used 1 to 4 times a day, the exact dose depending on the age, weight, and condition of the patient and on the frequency and route of administration. For the above use these compounds can be combined advantageously with other anti-asthmatic agents, such as sympathomimetics (isoproterenol, phenylephrine, ephedrine, etc.); xanthine derivatives (theophylline and aminophylline); and corticosteroids (ACTH and prednisolone).

As noted above, the compounds of this invention are particularly useful in treating asthma, but any allergy wherein slow reacting substance of anaphylaxis (SRSA) is thought to be involved as a pharmacological mediator of anaphylaxis can be treated. For example, the compounds can be used for treatment of such conditions as allergic rhinitis, food allergy and urticaria as well as asthma.

The compounds of this invention are effectively administered to human asthma patients by any covenient route such as oral inhalation, aerosol inhalation, parenterally, (orally, intravenously, interperitoneally), transdermally, topically and the like.

The amino acyl compounds of this invention are preferred for intravenous infusions and the like. Particularly preferred in this regard is L-Valine, 2-n-butyl-4-methoxy-1-naphthalenyl ester, monohydrochloride. The non-acyl-amino compounds of this invention are preferred for topical administration.

For administration by the oral inhalation route with conventional nebulizers or by oxygen aerosolization it is convenient to provide the instant active ingredient in dilute solution, preferably at concentrations of about 1 part of medicament to form about 100 to 200 parts by weight of total solution. Entirely conventional additives may be employed to stabilize these solutions or to provide isotonic media, for example, sodium chloride, sodium citrate, citric acid, sodium bissulfite, and the like can be employed.

For administration as a self-propelled dosage unit for administering the active ingredient in aerosol form suitable for inhalation therapy the composition can comprise the active ingredient suspended in an inert propellant (such as a mixture of dichlorodifluoromethane and dichlorotetrafluoroethane) together with a co-solvent, such as ethanol, flavoring materials and stabilizers. Instead of a co-solvent there can also be used a dispensing agent such as oleyl alcohol. Suitable means to employ the aerosol inhalation therapy technique are described fully in U.S. Pat. No. 2,868,691 for example.

The lipoxygenase inhibitor compounds of this invention are useful whenever it is desired to inhibit platelet aggregation, reduce the adhesive character of platelets, and remove or prevent the formation of

thrombi in mammals, including man, rabbits, dogs, and rats. For example, these compounds are useful in the prevention of myocardial infarcts, to prevent post-operative thrombosis, to promote patency of vascular grafts following surgery, and to treat conditions such as atherosclerosis, arteriosclerosis, blood clotting defects due to lipemia, and other clinical conditions. For these purposes, these compounds are administered systemically, e.g., intravenously, subcutaneously, intramuscularly, and in the form of sterile implants for prolonged action. For rapid response, especially in emergency situations, the intravenous route of administration is preferred. Doses in the range about 0.005 to about 20 mg per kg of body weight per day are used, the exact dose depending on the age, weight, and condition of the patient or animal, and on the frequency and route of administration.

These lipoxygenase inhibitor compounds are further useful as additives to blood, blood products, blood substitutes, or other fluids which are used in artificial extracorporeal circulation or perfusion of isolated body portions, e.g., limbs and organs, whether attached to the original body, detached and being preserved or prepared for transplant, or attached to a new body. During these circulations and perfusions, aggregated platelets tend to block the blood vessels and portions of the circulation apparatus. This blocking is avoided by the presence of these compounds. For this purpose, the compound is added gradually or in single or multiple portions to the circulating blood, to the blood of the donor animal, to the perfused body portion, attached or detached, to the recipient, or to two or all of these at a total steady state dose of about 0.001 to 10 mg per liter of circulating fluid. It is especially useful to use these compounds in laboratory animals, e.g., cats, dogs, rabbits, monkeys, and rats, for these purposes in order to develop new methods and techniques for organ and limb transplants.

Hammerström, et al. Science 197:994-996 (1977) notes the role of 12- lipoxygenase in psoriasis. Doig, et al., Prostaglandins 20:1007-1019 (1980) and Lin, et al., J. Clin. Invest. 70:1058 (1982) disclose that 5-lipoxygenase inhibitors block platlet thrombus formation. Dawson, et al., in SRS-A and Leukotrienes, 219-226 (Wiley and Sons 1981) note that 5-lipoxygenase inhibitors block neutrophil "recruitment" during inflammatory diseases such as arthritis.

In addition, 5-lipoxygenase inhibitors prevent the production of slow-reacting substance of anaphylaxis (SRS-A), now known to be a mixture of leukotrienes. (Leukotrienes are synthesized using 5-lipoxygenase.) SRS-A mediates the symptoms and pathophysiology of asthma. See Murphy, et al., Proc. Nat. Acad. Sci. USA, 4275-4279 (1979). Thus, the 5-lipoxygenase inhibitors disclosed herein are useful in the treatment of asthma.

5-Lipoxygenase products have been implicated in essential hypertension (Chand, et al., Microcirculation 1:111-123 (1981), and gout (Rae, et al., Lancet 1122-1124 (Nov. 20, 1982), indicating that the 5-lipoxygenase inhibitors disclosed herein are useful in treating these conditions as well. Further, neutrophil depletion, such as that induced by 5-lipoxygenase inhibitors, has been shown to cause a significant decrease in infarct size following circumflex artery occlusion. See Romson, et al., Circulation 66:85 (1982). Thus, the 5-lipoxygenase inhibitors herein may be useful in the protection of the myocardium following infarct.

The lipoxygenase inhibitors of the present invention are also useful for the prevention or treatment of deep vein thrombosis (DVT). This method comprises the administration of a compound of the Formula I to a mammal susceptible to DVT.

By "deep vein thrombosis" (DVT) is meant the thrombosis (clot formation) of the lower limb deep veins (deeply situated veins). Such thrombosis is frequently a result of major surgery, massive trauma, myocardial infarction, neoplasia, and pregnancy. The term "deep vein thrombosis" or "DVT" is meant to encompass the thrombosis resulting from these or any other causes.

By "prevention" in this context is meant the total or partial avoidance of clot formation in the deep veins of a mammal.

The present invention includes the treatment of each of various mammalian species, including humans. With respect to non-humans, the present invention is particularly and especially concerned with treating domesticated animals such as cattle, dogs, cats and swine. Nevertheless, compounds of the the invention are most preferably used in the treatment of humans.

Any convenient route of administration may be employed, such as parenteral (e.g. intravenous, intraperitoneal or intramuscular) administration, but oral administration is the preferred route for use in humans.

The dosage regimen for the lipoxygenase inhibitor compounds used to treat deep vein thrombosis will depend on a variety of factors, including the type, age, weight, sex and medical condition of the mammal, and most-importantly on the risks and probable consequences of deep vein thrombosis. A physician or veterinarian can determine these factors and prescribe an effective amount of the lipoxygenase inhibitor. When 1-acetoxy-2-butyl-4-methoxynaphthalene or 1-acetoxy-2,3-diethyl-4-methoxynaphthalene is used, the

dosage may be 0.01 to 1 mg/kg/min by intravenous infusion, or 0.01 to 50 mg/kg/day by oral administration. Equivalent dosages for other routes of administration may be used. When other lipoxygenase inhibitors are employed, equipotent doses are administered based on the compound's comparative potency as demonstrated in the standard laboratory test set forth as Example 20 below.

The most preferred used of these compounds is as SRS-A inhibitors, e.g. in the treatment of asthma.

Compounds of formula I (in which $R_3$ is H or $CH_3CO$) are prepared by the first reaction shown in Chart A. A carbene complex of formula A-1 is reacted with an alkyne of formula A-2 (preferably 1.5 equivalents). If $R_3$ is $CH_3CO$, the reaction may be carried out in the presence of acetic acid or acetic anhydride (and, preferably, triethylamine). The reaction is conducted with mild heating (preferably in a bath at 60 to 65 C) to yield the formula A-3 product. The reaction is preferably under in a solvent such as tetrahydrofuran (THF) in an inert atmosphere (e.g. argon).

Chromium complexes of formula A-1 are well known and readily available, or may be prepared by known means. Pentacarbonyl[aryl(methoxy)carbene]chromiums wherein the aryl moiety is phenyl, 2-(N-methylpyrrolyl), 2-furyl or 2-thienyl are described by Fisher, J. Organometal. Chem. 16 (1969) 29, Dotz et al., Chem. Ber. 111 (1978) 2517, Yamashita et al., Tet. Lett. 23 (1982) 3765, and Citilam et al., Inorg. Synth. 17 (1977); see also Preparations 1-4 below.

Similarly, alkynes of formula A-2 are well known, readily available compounds, or may be prepared by known means.

Compounds (amines) of formula I in which $R_3$ is other than H or $CH_3CO$, i.e. of formula Ia, are prepared from a corresponding compound (A-3) in which $R_3$ is OH, i.e. a phenolic compound, by reaction with an appropriate amino-acid of the formula $R_3OH$, e.g. of the formula

$HOOC-(CR_{17}R_{18})_m-(CH_2)_n-NR_{14}R_{15}$

(in which the amino group is either protected or non-protected), using the methodology of Hassner, Tet. Lett. (1978) 4475. Specifically, the phenolic compound is stirred at room temperature with the amino-acid and dicyclohexylcarbodiimide in a dry solvent (e.g. methylene chloride or ether) and in the presence of a catalyst (e.g. 4-dimethylaminopyridine or 4-pyrrolidinopyridine) under argon for 24 hours. When the amino group of the amin-acid is protected, the protecting group is removed by standard methodology. For example, a tert-butyloxycarbonyl (t-Boc) group is removed by using trifluoroacetic acid in dry methylene chloride, and a benzyl group is removed by catalytic hydrogenation.

The resultant amine is converted to a pharmacologically acceptable salt by treatment with an appropriate acid. The acids which are commonly used for salt formation include hydrochloric, hydrobromic, hydroiodic, methanesulfonic, ethanesulfonic, benzenesulfonic, phosphoric, acetic, propionic, succinic, para-toluenesulfonic, maleic, tartaric and lactic acids.

Compounds of the present invention in which $R_1$ and $R_2$ are ethyl or $R_2$ is n-butyl and $R_1$ is hydrogen are preferred. More preferred are compounds of this latter class when D is -CH=CH-. Still more preferred, because of their formulation characteristics, are compounds of this latter class, when $R_3$ is $-C(O)-(CR_{17}R_{18})_m-(CH_2)_n-NR_{14}R_{15}$, -C(O)AA, or $-C(O)-PhX-NH_2$. 1-Acetoxy-2-n-butyl-4-methoxynaphthalene (Example 3) and 1-acetoxy-2,3-diethyl-4-methoxy-naphthalene (Example 4) are more preferred compounds of this invention. L-Valine, 2,3-diethyl-4-methoxynaphth-1-yl ester, hydrochloride (Example 11), L-Valine, 2-n-butyl-4-methoxynaphth-1-yl ester, hydrochloride (Example 16), and L-Alanine, 2-n-butyl-4-methoxynaphth-1-yl ester, hydrochloride (Example 17) are the most preferred compounds of this invention.

DESCRIPTION OF THE PREFERRED EMBODIMENTS

The present invention is seen more fully by the Examples given below.

Preparation 1 Preparation of Pentacarbonyl[2-furyl(methoxy)carbene]chromium

A 500 ml two-necked round-bottomed flask, which is equipped with a stirring bar, a septum cap and an argon-vacuum inlet, is evacuated and filled with argon three times. Freshly distilled tetrahydrofuran (100 ml) and furan (7.5 ml, 0.1 mole) are placed in the flask, which are then cooled at -78° C with a dry ice-acetone bath. n-Butyl lithium (62 ml, 1.60 mmole/ml, n-hexane solution) is introduced via syringe to the furan solution slowly over a period of 15-20 minutes at -78° C. The resulting solution is warmed to -20° C, and stirred at this temperature for 15 hours (overnight) under argon. The 1000 ml three-necked round bottomed flask is equipped with a stirring bar, a septum cap and an argon-vacuum inlet. Chromium hexacarbonyl (22 g, 0.1 mole) is placed in this flask, which is evacuated and filled with argon three times. Dry ether (200 ml)

is added, and to this suspension is added the cooled (-20° C) solution of 2-lithiofuran at room temperature. During the procedure, chromium hexacarbonyl is dissolved and the solution turns to a deep red color. The resulting solution is stirred at room temperature 1 hour under argon, then concentrated using a rotary evaporator until the solution becomes a tarry residue (during concentration, the bath temperature is kept below 40° C). The deep red residue is dissolved in 200 ml water and trimethyloxonium tetrafluoroborate (-$(CH_3)_3O.BF4$) (solid) is added to the aqueous solution until it becomes slightly acidic (about pH ≈ 5.5) (15 g of $(CH_3)_3O.BF4$ is used). The aqueous layer is extracted three times with 250 ml of ether, and the combined extracts are washed once with 300 ml of saturated brine, once with 300 ml of saturated sodium carbonate aqueous solution, and again three times with 300 ml of saturated brine, dried over anhydrous sodium sulfate under argon atmosphere, and filtered. The solvent is removed using a rotary evaporator to give a deep red oil, which is dissolved in a small amount of methylene chloride and loaded on a silica gel column (200 g) using a flash chromatography apparatus. Separation of the products by column chromatography is carried out under a nitrogen atmosphere. Elution by 5% ether in n-hexane gives 27.5 g (90.9%) of pentacarbonyl[2-furyl(methoxy)carbene]chromium as deep red crystalline and elution by 20% ether in n-hexane gives 0.9 g (24%) of pentacarbonyl[2-furyl(methoxybutoxy)carbene]chromium as a deep red oil.

The physical properties of both products are as follows:

Pentacarbonyl[2-furyl(methoxy)carbene]chromium

Mass spectra (m/e): 302, 274, 246, 218, 190, 162, 147, 132, 119, and 52.
IR ($cm^{-1}$): 2060, 1992, 1443, 1386, 1224, and 1218.
$^1$H-NMR (δ, $CDCl_3$): 7.82, 7.02, 6.97, 6.60, 6.56, and 4.81.

Anal. Calc'd. for $C_{11}H_6O_7Cr$: C, 43.72; H, 2.00.
Found : C, 44.11; H, 2.17.

Pentacarbonyl[2-furyl(methoxybutoxy)carbene]chromium

Mass spectra (m/e): 374, 318, 290, 262, 234, 178, 148, and 52.
$^1$H-NMR: 7.83, 6.99, ~6.94, 6.59, 6.54, 5.11, 3.43, 3.35, and 2.10~1.70.

Preparation 2 Preparation of Pentacarbonyl[2-Thienyl(methoxy)carbene]chromium

A 500 ml two-necked round-bottomed flask, which is equipped with a stirring bar, a septum cap and an argon-vacuum inlet, is evacuated and filled with argon three times. Freshly distilled tetrahydrofuran (100 ml) and thiophene (8.01 ml) are placed in the flask, which is cooled to -78° C with a dry ice-acetone bath. n-Butyl lithium (62 ml, 1.60 mmole/ml, n-hexane solution) is introduced via syringe to the thiophene solution slowly over a period of 20 minutes at -78° C. The resulting solution is stirred at -30° C for 10 hours under argon. The 1000 ml three-necked round-bottomed flask is equipped with a stirring bar, a septum cap and an argon-vacuum inlet, and chromium hexacarbonyl (22 g, 0.1 mole) is placed in this flask, which is evacuated and filled with argon three times. Dry ether (200 ml) is introduced to the flask. A solution of 2-lithiothiophene (-30° C) is introduced via syringe to the suspension of chromium hexacarbonyl in ether at room temperature. During this procedure most of chromium hexacarbonyl is dissolved and the solution turns to a deep red color. The resulting solution is stirred at room temperature for 1 hour under argon, and concentrated using a rotary evaporation (bath temperature is kept below 40° C) until the solution becomes a thick tarry residue. The black residue is dissolved in 200 ml water and $(CH_3)_3O.BF4$ (solid) is added to the solution until it becomes slightly acidic (about pH ≈ 5.5). The aqueous solution is extracted three times with 250 ml of ether and the extracts are combined, washed once with 300 ml of saturated brine, once with 300 ml of saturated sodium bicarbonate aqueous solution and three times with 300 ml of saturated brine, and dried over anhydrous sodium sulfate under a stream of argon. The solution is filtered and the solvent is removed using a rotary evaporator (the bath temperature is kept below 40° C). The dark red oily residue is dissolved in a small amount of methylene chloride and loaded on a silica gel column (200 g) using flash chromatography apparatus under a nitrogen atmosphere. Elution by 5% ether in n-hexane gives 25.8 g (81.0%) of deep red crystals of pentacarbonyl[2-thienyl(methoxy)carbene]chromium and 1.22 g (3.13%) of pentacarbonyl[2-thienyl(methoxybutoxy)carbene]chromium as deep red crystals.

The physical properties of both products are as follows:

Pentacarbonyl[2-thienyl(methoxy)carbene]chromium

Mass spectra (m/e): 318, 290, 262, 234, 206, 178, 163, 148, 135, and 52.
IR ($cm^{-1}$): 2056, 1991, 1402, 1352, and 1238.

$^1$H-NMR (δ, $CDCl_3$): 8.28, 8.22, 7.73, 7.67, 7.23, 7.18, and 4.83.

Anal. Calc'd. for $C_{11}H_6O_6SCr$: C, 41.52; H, 1.90; S, 10.08.
Found : C, 41.81; H, 2.04; S, 9.25.

Pentacarbonyl[2-thienyl(methoxybutoxy)carbene]chromium

Mass spectra (m/e) calc'd. for $C_{15}H_{14}O_7SCr$: 389.987.
Found: 389.985.

IR ($cm^{-1}$): 2059, 1989, 1407.
$^1$H-NMR (δ, $CDCl_3$): 8.28, 8.23, 7.71, 7.65, 7.25 ~ 7.15, 5.14, 3.49, 3.36, and 2.25 ~ 1.70.

Anal. Calc'd. for $C_{15}H_{14}O_7SCr$: C, 46.15; H, 3.61; S, 8.22.
Found: C, 46.72; H, 3.68; S, 7.71.

Preparation 3 Preparation of Pentacarbonyl[phenyl(methoxy)carbene]chromium

To a suspension of 22 g (0.1 mole) of chromium hexacarbonyl in ether is slowly added phenyllithium (51 ml, 0.1 mole, cyclohexane:ether solution) via syringe over a period of 15-20 minutes under argon at room temperature, and the resulting deep red solution is stirred at room temperature for 1 hour. The solvent is removed under reduced pressure (bath temperature should be below 40° C), and the black residue is dissolved in 200 ml water. $(CH_3)_3O.BF4$ (about 15 g) is added portionwise to the solution until it becomes slightly acidic (pH ≈ 5.5). The aqueous layer is extracted three times with 200 ml of ether, and the combined etheral extracts are washed once with 300 ml of saturated brine solution, once with 300 ml of saturated sodium carbonate solution, and three times with 300 ml of saturated brine solution, dried over anhydrous sodium sulfate and filtered. The solvent is removed using a rotary evaporator and the deep red tarry residue is purified by a silica gel column (200 g) using flash chromatography. Elution by ~ 10% ether in n-hexane gives a deep red syrup, which is solidified upon cooling. Recrystallization from pet-ether at -70° C gives 25.12 g (80.5%) of pentacarbonyl[phenyl(methoxy)carbene]chromium as deep red crystalline.

The physical properties of the product are consistent with those described in the literature.

Preparation 4 Preparation of Pentacarbonyl[2-N-methylpyrrolyl(methoxy)carbene]chromium

A 500 ml two-necked round-bottomed flask, which is equipped with a stirring bar, a septum cap and an argon-vacuum inlet, is evacuated and filled with argon three times. Tetrahydrofuran (100 ml) and N-methylpyrrole (9.0 ml, 0.1 mole) are placed in this flask, which is then cooled at -78° C with dry ice-acetone bath. n-Butyllithium (62 ml, 1.60 mmole/1 ml n-hexane solution) is introduced via syringe to the pyrrole solution slowly over a period of 15 minutes at -78° C. The resulting pale yellow solution is warmed to -20° C, and stirred at this temperature for 15 hours under argon. A 1000 ml three-necked round-bottomed flask is equipped with a stirring bar, a septum cap and an argon-vacuum inlet, and chromium hexacarbonyl (22 g, 0.1 mole) is placed in this flask, which is evacuated and filled with argon three times. Dry ether (200 ml) is added and to this suspension is added the cooled (-20° C) solution of 2-lithio-N-methylpyrrole at room temperature via syringe. During this procedure, most of the chromium hexacarbonyl is dissolved and the solution is stirred at room temperature for 1 hour under argon, and then concentrated using a rotary evaporator until the solution becomes a tarry residue (during concentration, bath temperature should be below 40° C). The deep red residue is dissolved in 200 ml water and $(CH_3)_3O.BF4$ (solid) is added to the solution until it becomes slightly acidic (about pH ≈ 5.5) (15 g of $(CH_3)_3O.BF4$ is used). The aqueous solution is extracted three times with 250 ml of ether, and the combined extracts are washed once with 300 ml of saturated brine, once with 300 ml of saturated sodium carbonate, and three times with 300 ml of saturated brine, and finally dried over anhydrous sodium sulfate under argon atmosphere and filtered. The solvent is removed using a rotary evaporator (bath temperature should be below 40° C). The dark red

residue is dissolved in a small amount of methylene chloride, and loaded on a silica gel column (200 g) using flash chromatography apparatus. Separation of the product by column chromatography is carried out under nitrogen atmosphere. Elution by 10% ether in n-hexane gives a small amount of pentacarbonyl[n-butyl(methoxy)carbene]chromium as a yellow oil, elution by 40% ether in n-hexane gives 19.63 g (62.3%) of pentacarbonyl[2-N-methylpyrrole(methoxy)carbene]chromium as orange crystals. Further elution by a 1:1 mixture of ether and n-hexane gives a small amount of pentacarbonyl[2-N-methylpyrrole(methoxybutoxy)-carbene]chromium as a deep red oil.

The physical properties of the desired product are as follows:
Mass spectra (m/e): 315, 287, 259, 231, 203, 175, 160, 145, 132, and 52.
IR ($cm^{-1}$): 2053, 1984, 1400, 1343, and 1323.
$^1$H-NMR ($\delta$, $CDCl_3$): 7.75, 7.70, 6.79, 6.29, 6.27, 4.71, and 3.73.

Anal. Calc'd. for $C_{12}H_9NO_6Cr$: C, 45.75; H, 2.88; N, 4.44.
Found : C, 45.82; H, 3.02; N, 4.38.

Example 1 5-Butyl-7-methoxy-4-benzofuranol (Formula I: D is -O-, $R_3$ is hydrogen, $R_2$ is n-butyl, and $R_1$ is hydrogen)

Reaction of pentacarbonyl[2-furyl(methoxy)carbene]chromium with 1-hexyne.

A mixture of 1.0 g (3.3 mmole) of the carbene complex and 1 ml (2.6 eq) of 1-hexyne in tetrahydrofuran is heated at 65°C for 6 hours under argon atmosphere. After cooling the solvent is removed and the product is purified by flash column chromatography (silica gel), eluting with 5% ether in n-hexane, to give 552 mg (76.0%) of the title product as a colorless oil, which solidifies upon cooling.

Physical characteristics of the product are as follows:
Mass spectra (m/e): 220, 205, 177, and 163.
IR ($cm^{-1}$): 3200, 1634, 1465, and 1346.
$^1$H-NMR ($\delta$, $CDCl_3$): 7.52, 6.77, 6.56, 4.65, 3.95, 2.80~2.50, and 1.90~0.85.

Anal. Calc'd. for $C_{13}H_{16}O_3$: C, 70.88; H, 7.32.
Found : C, 71.01; H, 7.36.

Example 2 5-Butyl-7-methoxy-benzo(b)thiophen-4-ol (Formula I: D is -S-, $R_3$ is hydrogen, $R_2$ is n-butyl, and $R_1$ is hydrogen)

Reaction of pentacarbonyl[2-thienyl(methoxy)carbene]chromium with 1-hexyne.

A solution of 200 mg (0.62 mmole) of the carbene complex and 1-hexyne (1.5 eq) in tetrahydrofuran, prepared under argon, is heated at 60°C for 4 hours. After the solution is cooled to room temperature, the reaction mixture is stirred at room temperature overnight under argon. The solvent is removed under reduced pressure, and the residue is loaded on a silica gel plate (1500 micron) for thick layer chromatography. Developing by n-hexane:ether (4:1 mixture) three times yields 64 mg (43.8%) of the title product.

The physical characteristics of the products are as follows:
Mass spectral (m/e): 236, 221, 193, and 179.
IR ($cm^{-1}$): 3282, 1579, 1516, 1465, and 1447.
$^1$H-NMR ($\delta$, $CDCl_3$): 7.50~7.20, 6.54, 3.93, 2.75~2.50, and 2.00-0.75.

Anal. Calc'd. for $C_{13}H_{16}O_2S$: C, 66.07; H, 6.83; S, 13.57.
Found : C, 65.70; H, 6.86; S, 13.19.

Example 3 2-Butyl-4-methoxy-1-napthalenol, acetate (Formula I: D is -CH=CH-; $R_3$ is $CH_3$-C(O)-; $R_2$ is n-butyl, and $R_1$ is hydrogen)

Reaction of pentacarbonyl[phenyl(methoxy)carbene]chromium with 1-hexyne.

Part A

A mixture of the carbene complex (1.0 g, 3.2 mmole), 1-hexyne (2.6 eq), acetic anhydride (1.0 eq) and triethylamine (1.0 eq) in tetrahydrofuran (90 ml) is heated at 65° C under argon atmosphere for 1 hour. The solution is cooled and concentrated to give a black residue which is chromatographed through a silica gel (200 g) column using a flash chromatography. Elution by 10% ether in n-hexane gives 715 mg (82.2%) of the title product which solidified. Recrystalization from pet-ether gave white crystals, mp 49° C

The physical properties are as follows:
Mass spectra (m/e): 272, 230, and 187.

Calc'd. for $C_{17}H_{20}O_3$: 272.1412
Found : 272.1412.

IR ($cm^{-1}$): 1761, 1597, 1370, 1202, 1162, and 765.
$^1H$-NMR (δ, $CDCl_3$): 8.25~8.10, 7.75~7.40, 6.65, 3.98, 2.75-2.50, 2.45, and 1.80-0.80.

Anal. Calc'd. for $C_{17}H_{20}O_3$: C, 74.97; H, 7.41.
Found : C, 75.02; H, 7.40.

Part B

Alternatively, a mixture of 2.0 g (6.4 mmole) of the carbene complex, acetylene (2.6 eq) and acetic acid in tetrahydrofuran is heated at 65° C for 2 hours under an argon atmosphere. After cooling the reaction solution is concentrated and the black residue is loaded on a silica gel (200 g) column for a flash chromatography. Elution by 10% ether in n-hexane gives 895 mg (51.4%) of the title product.

The physical properties of the product is the same as described above.

Example 4 2,3-Diethyl-4-methoxy-1-naphthalenol, acetate (Formula I: D is -CH=CH-, $R_3$ is $-CH_3-C(O)-$, $R_2$ and $R_1$ are ethyl)

Reaction of pentacarbonyl[phenyl(methoxy)carbene]chromium with 3-hexyne in the presence of acetic anhydride and triethylamine.

A mixture of the carbene complex (2.0 g, 6.4 mmole), 3-hexyne (2.0 ml, 2.8 eq), acetic anhydride (0.65 ml, 1.1 eq) and triethylamine (0.9 ml, 1.0 eq) in tetrahydrofuran (180 ml) is heated at 65° C for 2 hours under argon atmosphere. After cooling the solvent is removed and the black residue is loaded on silica gel column for 74.7% of the title product as a yellow oil.

Physical characteristics are as follows:
Mass spectra (m/e): 272, 230, and 215.

Calc'd. for $C_{17}H_{20}O_3$: 272.1412
Found : 272.1412.

IR ($cm^{-1}$): 1762, 1594, 1357, 1208, and 1096.
$^1H$-NMR (δ, $CDCl_3$): 8.10-7.85, 7.70-7.30, 3.94, 2.47, 2.80-2.50, 1.25, and 1.20.

Anal. Calc'd. for $C_{17}H_{20}O_3$: C, 74.97; H, 7.40.
Found : C, 75.35; H, 7.56.

Example 5 4-Methoxy-2,3-diphenyl-1-naphthalenol, acetate (Formula I: D is -CH=CH-, $R_3$ is $CH_3C(O)$-, $R_2$ and $R_1$ are phenyl)

Reaction of pentacarbonyl[phenyl(methoxy)carbene]chromium with diphenylacetylene in the presence of acetic anhydride and triethylamine.

A mixture of the carbene complex (1.0 g, 3.2 mmole), acetylene (1.0 g, 1.8 eq), acetic anhydride (0.3 ml, 1.0 eq) and triethylamine (0.45 ml, 1.0 eq) in 90 ml of tetrahydrofuran is heated at 65° C for 2 hours under an argon atmosphere. After cooling, the reaction solution is concentrated to give a black residue, which is chromatographed through a silica gel (200 g) column using a flash chromatography. Elution by 20% ether in n-hexane gives 620 mg (53.0%) of the title product as a yellow oil.

Physical characteristics are as follows:

Mass spectra (m/e): 368, 326, and 311.

Calc'd. for $C_{25}H_{20}O_3$: 368.1412.
Found : 368.1422.

IR ($cm^{-1}$): 1768.

$^1$H-NMR (δ, $CDCl_3$): 8.25~8.00, 7.80~7.40, 7.15, 3.51, and 2.04.

Anal. Calc'd. for $C_{25}H_{20}O_3$: C, 81.40; H, 5.47.
Found : C, 81.10; H, 5.60.

Example 6 5-Butyl-7-methoxy-1-methyl-1H-Indol-4-ol, acetate (ester) (Formula I: D is =$N(CH_3)$; $R_3$ is $CH_3C(O)$-, $R_2$ is n-butyl, $R_1$ is hydrogen).

Reaction of pentacarbonyl[2-(N-methyl)pyrrolyl(methoxy)carbene]chromiumwith 1-hexyne in the presence of acetic anhydride and triethylamine.

A mixture of the carbene complex (2.0 g, 6.4 mmole), acetylene (2 ml, 2.6 eq , acetic anhydride, (0.7 ml, 1.1 eq) and triethylamine (0.9 ml, 1.1 eq) in 180 ml of tetrahydrofuran is heated at 65° C under an argon atmosphere for 1-2 hours. The solution is cooled and concentrated. The residue is loaded on a silica gel (480 ml) column for flash chromatography, yielding the title product as a brown oil.

Physical characteristics are as follows:

Mass spectra (m/e): 275, 233, and 190.

IR ($cm^{-1}$): 1759, 1581, 1502, 1207, and 1013.

$^1$H-NMR (δ, $CDCl_3$): 6.84, 6.39, 6.14, 3.97, 3.88 2.60~2.30, 2.36, and 1.75~0.75.

Anal. Calc'd. for $C_{16}HH_{21}NO_3$: C, 69.79; H, 7.69; N, 5.09.
Found : C, 70.00; H, 7.81; N, 5.00.

Example 7 5,6-Diethyl-7-methoxy-1-methyl-1H-indol-4-ol, acetate (ester) (Formula I: D is =$N(CH_3)$; $R_3$ is $CH_3C(O)$-, $R_2$ and $R_1$ are ethyl)

Reaction of pentacarbonyl[2-(N-methyl)pryyolyl(methoxy)carbene] chromium with 3-hexyne in the presence of acetic anhydride.

Part A

A mixture of the carbene complex (2.5 g, 7.9 mmole), 3-hexyne (2.5 ml, 2.8 eq) and acetic anhydride (0.75 ml, 1.1 eq) in 200 ml of tetrahydrofuran is heated at 60~65° C for 5 hours under argon. The reaction solution is cooled and concentrated. The black residue (one spot on TLC) is chromatographed through a silica gel (220 g) column using a flash chromatography. Elution by 25% ether in n-hexane gives 2.17 g

(42.4%) of the title product as a yellow oil.

The physical characteristics are as follows:

Mass spectra (m/e): 275, 233, and 218.

Calc'd. for $C_{16}H_{21}NO_3$: 275.1521.
Found : 275.1515.

IR ($cm^{-1}$): 1761, 1488, 1295, 1216, and 1196.

$^1$H-NMR (δ, $CDCl_3$): 6.84, 6.14, 3.97, 3.85, 2.78-3.62, 1.22, and 1.44.

Anal. Calc'd. for $C_{16}H_{21}NO_3$: C, 69.79; H, 7.69; N, 5.09.
Found : C, 69.60; H, 7.46; N, 5.10.

Part B

Alternatively, a mixture of 1.5 g (4.8 mmole) of the carbene complex, 1.5 ml of 3-hexyne, 0.9 ml of acetic anhydride and 0.7 ml of triethylamine in 130 ml of tetrahydrofuran is heated at 65° C for 6 hours under a 1.3 ml argon atmosphere. The reaction is carried out in the same manner described above. Silica gel flash column chromatography yields 590 mg (44.7%) of the title product.

Example 8 7-Methoxy-1-methyl-5,6-diphenyl-1H-indol-4-ol, acetate (ester) (Formula I: D is $=N(CH_3)$; $R_3$ is $CH_3C(O)-$; $R_1$ and $R_2$ are phenyl)

Reaction of pentacarbonyl[2-(N-methyl)pyrrolyl(methoxy)carbene]chromiumwith diphenyl acetylene in the presence of acetic anhydride and triethylamine.

A mixture of the carbene complex (1.5 g, 4.8 mmole), acetylene (1.8 eq), acetic anhydride (2.0 eq) and triethylamine (1.0 eq) in tetrahydrofuran (150 ml) is heated at 65° C under argon atmosphere for 5 hours. After cooling, the solvent is removed and the product is purified by a silica gel flash column chromatography. Elution by 50% ether in n-hexane gives 390 mg (22.0%) of the title product as yellow crystals.

The physical properties are as follows:

Melting point: 158~159.5° C.

Mass spectra (m/e): 371, 329, and 314.

Calc'd. for $C_{24}H_{21}NO_3$: 371.1521
Found : 371.1511

IR ($cm^{-1}$): 1761, 1599, and 1202.

$^1$H-NMR (δ, $CDCl_3$): 7.13, 6.99, 6.32, 4.03, 3.36, and 2.01.

Anal. Calc'd. for $C_{24}H_{21}NO_3$: C, 77.61; H, 5.70; N, 3.77.
Found : C, 77.27; H, 5.73; N, 3.67.

Example 9 5-Butyl-7-methoxy-4-benzofuranol, acetate (Formula I: D is -O-, $R_3$ is $CH_3C(O)-$, $R_2$ is n-butyl, $R_1$ is hydrogen)

Reaction of pentacarbonyl[2-furyl(methoxy)carbene]chromium with 1-hexyne in the presence of acetic anhydride and triethylamine.

A mixture of the carbene complex (1 g, 3.3 mmole), 1-hexyne (0.57 ml, 1.5 eq), acetic anhydride (1.0 eq) and triethylamine (1.0 eq) in tetrahydrofuran (90 ml), prepared under argon atmosphere, is heated at

65° C under argon for 6 hours. An additional 1-hexyne (1.0 eq) is added to the reaction mixture, which is heated at 65° C for an additional 17 hours. The resulting black solution is cooled and the solvent is removed by using a rotary-evaporator. The black residue is chromatographed through flash column chromatography (250 g of silica gel). Elution by 30% ether in n-hexane yields 590 mg (68.2%) of the title product as a pale purple solid.

Physical characteristics are as follows:
Melting point: 49-50° C.
Mass spectra (m/e): 262, 220, and 177.

Calc'd. for $C_{15}H_{18}O_4$: 262.1205.
Found : 262.1211.

IR ($cm^{-1}$): 1761, 1490, 1446, 1349, and 1208.
$^1$H-NMR (δ, $CDCl_3$): 7.54, 6.63, 6.55, 3.99, 2.75~2.45, 2.37, and 1.75~0.80.

Anal. Calc'd. for $C_{15}H_{18}O_4$: C, 68.69; H, 6.92.
Found : C, 68.80; H, 7.00.

Example 10 5-Butyl-7-methoxy-benzo(b)thiophen-4-ol, acetate (Formula I: D is -S-; $R_3$ is $CH_3C(O)$-; $R_2$ is n-butyl; $R_1$ is hydrogen)

Reaction of pentacarbonyl[2-thienyl(methoxy)carbene]chromium with 1-hexyne in the presence of acetic anhydride and triethylamine.

A mixture of the carbene complex (1.0 g, 3.1 mmole), 1-hexyne (0.53 ml, 1.5 eq), acetic anhydride (1.0 eq), and triethylamine (1.0 eq) in tetrahydrofuran (90 ml), prepared under an argon atmosphere, is heated at 65° C under argon. After heating at this temperature for 4 hours, an additional 0.26 ml (1.0 eq) of 1-hexyne is introduced to the reaction mixture, which is heated at 65° C under argon for an additional 16 hours. The resulting black solution is cooled, and concentrated to dryness by aid of a rotary evaporator. The black residue is chromatographed by using flash column chromatography (300 g of silica gel). Elution by 15% ether in n-hexane gives 570 mg (66.1%) of the title product as a pale yellow solid.

Physical characteristics are as follows:
Melting point: 51~52° C (recrystallized from cold pet ether/n-hexane).
Mass spectra (m/e): 278, 236, and 193.
IR ($cm^{-1}$): 1762, 1510, 1465, 1436, 1375, and 1202.
$^1$H-NMR (δ, $CDCl_3$): 7.36, 7.13, 6.61, 3.96, 2.39, 2.75~2.40, and 1.80~0.80.

Anal. Calc'd. for $C_{15}H_{18}O_3S$: C; 64.49; H, 6.55; S, 11.52.
Found : C, 64.75; H, 6.55; S, 11.69.

Example 11 L-Valine, 2,3-diethyl-4-methoxynaphth-1-yl ester and its hydrochloric acid salt (Formula I: D is -CH=CH-, $R_3$ is $-C(O)-(CR_{17}R_{18})_m-(CH_2)_n-NR_{14}R_{15}$, $R_2$ is ethyl, $R_1$ is ethyl, $R_{17}$ is hydrogen, $R_{18}$ is isopropyl, $R_{14}$ is hydrogen, $R_{15}$ is hydrogen, n is 0, and m is 1).

Part A

Dry methylene chloride (15 ml) is introduced to a mixture of 2,3-diethyl-4-methoxynaphthol (100 mg, 0.44 mmole), t-Boc-L-Valine (2 eq, 189 mg, 0.88 mmole), dicyclohexylcarbodiimide (2 eq, 180 mg, 0.88 mmole), and 4-dimethylaminpyridine (1 eq, 55 mg, 0.44 mmole) at room temperature under an argon atmosphere. The resulting solution is stirred at room temperature under argon for 20 hours, then diluted with 100 ml of methylene chloride. The white precipitate is removed by filtration, and the filtrate washed once with 50 ml of water, twice with 50 ml of saturated aqueous sodium bicarbonate solution, and then three times with 50 ml of water. The extracts are dried ov r anhydrous sodium sulfate, filtered and

concentrated. Thick layer chromatography (silica gel), developed by 1:4 mixture of ether and n-hexane, isolates 200 mg (quantitative yield) of the t-Boc-L-Valine,2,3-diethyl-4-methoxynaphth-1-yl ester as a colorless syrup.

Physical characteristics are as follows:
$^1$H-NMR (δ, $CDCl_3$): 8.10-7.90, 7.70-7.50, 7.50-7.30, 5.20-4.90, 4.70-4.50, 3.93, 3.00-2.50, 1.49, and 1.40-1.00.

Part B

Trifluoroacetic acid (50 ml) is added slowly to an ice bath solution of t-Boc-L-Valine,2,3-diethyl-4-methoxynaphth-1-yl ester (2.68 g, 6.2 mmole) in dry methylene chloride (70 ml) under argon. The ice bath is removed and the resulting yellow solution stirred at room temperature for 1 hour under argon. The solvents are removed using a rotary evaporator and the residue is dissolved in methylene chloride. The methylene chloride extracts are washed with an aqueous sodium bicarbonate solution once and three times with 200 ml of water, dried over anhydrous sodium sulfate, filtered and concentrated. The residue is chromatographed by using a silica gel (200 g) flash column and eluted by 10% methanol in methylene chloride, giving 2.09 g of L-Valine,-2,3-diethyl-4-methoxynaphth-1-yl ester (quantitative yield) as a colorless oil.

Physical characteristics are as follows:
Mass spectra (m/e): 329, 230, 214, and 72.
IR($cm^{-1}$): 3394, 1755, 1359, 1133.
$^1$H-NMR (δ, $CDCl_3$): 8.15-7.90, 7.70-7.30, 3.94, 3.86, 3.00-2.45, and 1.40-1.00.

Anal. Calc'd. for $C_{20}H_{27}NO_3$: 329.1991.
Found : 329.1993.

The deprotected L-Valine,2,3-diethyl-4-methoxynaphth-1-yl ester is dissolved in a small amount of dry methylene chloride and treated with hydrochloric acid saturated anhydrous ether. Recrystallization from ether in n-hexane gives the hydrochloric acid salt.

Physical characteristics are as follows:
Melting point: 183-186 °C.
Mass spectra (m/e): 329, 230, 215, and 72.
IR ($cm^{-1}$): 1761, 1463, 1357, and 1191.

Anal. Calc'd. for $C_{20}H_{28}NO_3Cl$: C, 65.65; H, 7.71: N, 3.83; Cl, 9.69.
Found : C, 65.70; H, 8.01; N, 3.78; Cl, 9.54.

Example 12 L-Alanine, 2,3-diethyl-4-methoxynaphth-1-yl ester (Formula I: D is -CH=CH-, $R_3$ is -C(O)-$(CR_{17}R_{18})_m$-$(CH_2)_n$-$NR_{14}R_{15}$, $R_2$ is ethyl, $R_1$ is ethyl, $R_{17}$ is hydrogen, $R_{18}$ is methyl, $R_{14}$ is hydrogen, $R_{15}$ is hydrogen, n is 0, and m is 1).

Part A

Dry methylene chloride (35 ml) is added to a mixture of 2,3-diethyl-4-methoxynaphthol (200 mg, 0.87 mmole), t-Boc-L-Alanine (2 eq, 326 mg, 1.74 mmole), dicyclohexylcarbodiimide (2 eq, 360 mg, 1.74 mmole) and 4-dimethylaminpyridine (1 eq, 110 mg, 0.87 mmole) at room temperature under an argon atmosphere. The resulting solution is stirred at room temperature under argon for 20 hours, then diluted with methylene chloride. The white precipitate is removed by filtration, and the filtrate is washed once with water, twice with saturated aqueous sodium bicarbonate solution and three times with water. The extracts are dried over anhydrous sodium sulfate, filtered and concentrated. Thick layer chromatography (2000 micron, silica gel), developed by 1:4 mixture of ether-n-hexane, isolates 326 mg (93.5%) of t-Boc-L-Alanine,

2,3-diethyl-4-methoxynaphth-1-yl ester as a colorless oil.

Physical characteristics are as follows:

Mass spectra (m/e): 401, 328, 230, and $^{+}$215.

$^1$H-NMR (δ, $CDCl_3$): 8.15-7.85, 7.75-7.55, 7.50-7.30, 5.20-5.00, 4.90-4.60, 3.93, 3.05-2.45, 1.70, 1.48, 1.24 and 1.18.

Part B

Trifluoroacetic acid (50 ml) is introduced slowly to a cooled ice bath solution of t-Boc-L-Alanine,2,3-diethyl-4-methoxynaphth-1-ylester (2.70 g, 6.70 mmole) in dry methylene chloride (70 ml) under argon. The ice bath is removed and the resulting yellow solution is stirred at room temperature for 1.5 hours under argon. The solvents are removed using a rotary evaporator and the residue is dissolved in 400 ml of methylene chloride. The methylene chloride extracts are washed once with aqueous sodium bicarbonate, three times with water, dried over anhydrous sodium sulfate, filtered and concentrated. The residue is chromatographed through a silica gel (200 g) flash column, and eluted by 2% methanol in methylene chloride to give 1.97 g (97.5%) of a yellow oil.

Physical characteristics are as follows:

Mass spectra (m/e): 301, 230, 215, and $^{+}$44.

IR ($cm^{-1}$): 3482, 1757, 1660, 1451, 1376, 1359, and 1153.

$^1$H-NMR (δ, $CDCl_3$): 8.20-7.95, 7.70-7.30, 4.05, 3.93, 3.00-2.50, 1.67, 1.18, and 1.15.

Anal. Calc'd. for $C_{18}H_{23}NO_3$: 301.1678.
Found : 301.1685.

Example 13 L-Phenylalanine, 2,3-diethyl-4-methoxynaphth-1-yl ester (Formula I: D is -CH=CH-, $R_3$ is -C-(O)-$(CR_{17}R_{18})_m$-$(CH_2)_n$-$NR_{14}R_{15}$, $R_2$ is ethyl, $R_1$ is ethyl, $R_{17}$ is hydrogen, $R_{18}$ is benzyl, $R_{14}$ is hydrogen, $R_{15}$ is hydrogen, n is 0, and m is 1).

Part A

Dry methylene chloride (100 ml) is introduced to a mixture of 2,3-diethyl-4-methoxynaphthol (1 g, 4.3 mmole), t-Boc-L-phenylalanine (1.5 eq, 1.7 g, 6.4 mmole), dicyclohexylcarbodiimide (1.5 eq, 1.4 g, 6.4 mmole), and 4-dimethylaminpyridine (0.37 eq, 0.2 g, 1.6 mmole) under an argon atmosphere at room temperature. The resulting solution is stirred at room temperature for 20 hours under argon. The white precipitates are removed by filtration and the filtrate washed twice with 100 ml of saturated aqueous sodium bicarbonate solution and three times with 100 ml of water, dried over anhydrous sodium sulfate, filtered and concentrated. The residue is chromatographed through a silica gel (200 g) flash column, and eluted by 10-15% ether in n-hexane to give 1.7 g of t-Boc-L-phenylalanine, 2,3-diethyl-4-methoxynaphth-1-ylester as a colorless amorphous crystal.

Physical characteristics are as follows:

Mass spectra (m/e): 404, 230, and $^{+}$215.

IR ($cm^{-1}$): 1762, 1716, 1689, 1455, and 1367.

$^1$H-NMR (δ, $CDCl_3$): 8.15-7.90, 7.60-7.30, 5.20-4.90, 3.93, 3.50-3.20, 3.00-2.40, 1.44, and 1.40-1.00.

Anal. Calc'd. for $C_{29}H_{35}NO_5$: C, 72.93; H, 7.39; N, 2.93.
Found : C, 72.63; H, 7.45; N, 2.88.

Part B

A solution of the t-Boc-L-phenylalanine,2,3-diethyl-4-methoxynaphth-1-yl ester (1.6 g, 3.4 mmole) in dry methylene chloride (40 ml), prepared under argon, is treated with trifluoroacetic acid (10 ml) at 0° C (ice bath), and the yellow solution stirred at 0° C for 30 minutes under argon. The ice bath is removed and the

solution is stirred at room temperature for an additional 45 minutes under argon. The solvents are removed using a rotary evaporator and the residue is dissolved in 300 ml of methylene chloride. The methylene chloride extracts are washed three times with 200 ml of water, dried over anhydrous sodium sulfate, filtered and concentrated. The residue is chromatographed through a silica gel (200 g) flash column and eluted by 1-2% methanol in methylene chloride to give 1.11 g (86.7%) of L-phenylalanine, 2,3-diethyl-4-methoxynaphth-1-yl ester as a pale yellow oil.

Physical characteristics are as follows:
Mass spectra (m/e): 377, 230, 215, 120, and 91.
IR ($cm^{-1}$): 3380, 3320, 3060, 3030, 2870, 2830, 2770, 1755, 1685, 1660, 1595, 1575, 1495, 1455, 1360, 1145, 1105, 1020, 760, 745, and 700.
$^1$H-NMR ($\delta$, $CDCl_3$): 8.15-7.85, 7.50-7.25, 4.30-4.05, 3.94, 3.50-2.95, 2.95-2.45, 1.24 and 1.16.

Anal. Calc'd. for $C_{24}H_{27}NO_3$: 377.1991.
Found : 377.1988.

In accordance with the procedure of Example 13, Part B, the free amine is converted to the hydrochloric acid salt and recrystallized from absolute ethanol and ether in n-hexane.

Physical characteristics are as follows:
Melting point: 218-200° C.
Mass spectra (m/e): 377, 249, 230, 215, and 120.
IR ($cm^{-1}$): 3060, 3030, 2720, 2630, 1755, 1595, 1575, 1510, 1495, 1200, 760, and 705.

Anal. Calc'd. for $C_{24}H_{28}ClNO_3$: C, 69.64; H, 6.82; N, 3.38; Cl, 8.57.
Found: C, 69.48; H. 6.63; N, 4.06; Cl, 8.03

Example 14 3-(N,N-Diethylamino) propionic acid, 2,3-diethyl-4-methoxynaphth-1-yl ester. (Formula I: D is -CH=CH-, $R_3$ is -C(O)-$(CR_{17}R_{18})_m$-$(CH_2)_n$-$NR_{14}R_{15}$, $R_2$ is ethyl, $R_1$ is ethyl, $R_{17}$ is hydrogen, $R_{18}$ is hydrogen, $R_{14}$ is ethyl, $R_{15}$ is ethyl, n is 1, and m is 1).

Dry methylene chloride (30 ml) and triethylamine (0.33 ml, 2.34 mmole) are added to a mixture of 2,3-diethyl-4-methoxynaphthol (180 mg, 0.78 mmole), dimethylamino propionic acid hydrochloride (2 eq, 283 mg, 1.56 mmole), dicyclohexylcarbodiimide (2 eq, 322 mg, 1.56 mmole) and 4-dimethylaminpyridine (1 eq, 95 mg, 0.78 mmole) at room temperature under argon, and the resulting suspension is stirred at room temperature under argon for two days. The white precipitate is removed by filtration, and the filtrate is concentrated. Thick layer chromatography (silica gel, 2000 micron) developed by a 9:1 mixture of ether and n-hexane isolates 144 mg (51.8%) of 3-(N,N-diethylamino) propionic acid, 2,3-diethyl-4-methoxynaphth-1-yl ester as a colorless oil.

Physical characteristics are as follows:
Mass spectra (m/e): 357, 284, 230, 215, and 86.
IR ($cm^{-1}$): 1758, 1660, 1595, 1451, and 1360.
1H-NMR ($\delta$, $CDCl_3$): 8.10-7.70, 7.50-7.30, 3.94, 3.00-2.45, and 1.40-0.90.

Anal. Calc'd. for $C_{22}H_{31}O_3N$: 357.2304.
Found : 357.2292.

Example 15 N,N-Dimethylamino acetic acid,2,3-diethyl-4-methoxynaphth-1-yl ester (Formula I: D is -CH=CH-, $R_3$ is -C(O)-$(CR_{17}$-$R_{18})_m$-$(CH_2)_n$-$NR_{14}R_{15}$, $R_2$ is ethyl, $R_1$ is ethyl, $R_{17}$ is hydrogen, $R_{18}$ is hydrogen, $R_{14}$ is methyl, $R_{15}$ is methyl, n is 0, and m is 1).

Triethylamine (3 eq) and dry methylene chloride (150 ml) are introduced to a mixture of 2,3-diethyl-4-methoxynaphthol (1.59 g, 6.5 mmole), dimethylamino acetic acid hydrochloride (2 eq, 1.8 g, 13.0 mmole), dicyclohexylcarbodiimide (2 eq, 2.7 g, 13.0 mmole) and 4-dimethylaminpyridine (1 eq, 2.7 ml, 19.5 mmole), prepared under an argon atmosphere, at room temperature under argon. The resulting suspension is stirred at room temperature for 20 hours under argon, then diluted with 300 ml of methylene chloride. The methylene chloride extracts are washed thoroughly with saturated aqueous sodium bicarbonate solution and water, dried over anhydrous magnesium sulfate, filtered and concentrated. The residue is chromatographed through a silica gel (200 g) flash column and eluted by ether to give 1.48 g (72.2%) of N,N-dimethylamino acetic acid, 2,3-diethyl-4-methoxynaphth-1-yl ester as a yellowish brown oil.

Physical characteristics are as follows:
Mass spectra (m/e): 315, 287, 229, and 58.
IR ($cm^{-1}$): 1775, 1595, 1452, 1358, and 1131.
$^1$H-NMR ($\delta$, $CDCl_3$): 8.20-7.90, 7.65-7.30, 3.93, 3.67, 3.00-2.50, 2.53, 1.24, and 1.19.

Anal. Calc'd. for $C_{19}H_{25}NO_3$: 315.1834.
Found : 315.1845.

Example 16 L-Valine, 2-n-butyl-4-methoxynaphth-1-yl ester and its hydrochloric acid salt (Formula I: D is -CH=CH-, $R_3$ is $-C(O)-(CR_{17}R_{18})_m-(CH_2)_n-NR_{14}R_{15}$, $R_2$ is butyl, $R_1$ is hydrogen, $R_{17}$ is hydrogen, $R_{18}$ is isopropyl, $R_{14}$ is hydrogen, $R_{15}$ is hydrogen, n is 0, and m is 1).

Part A

Dry methylene chloride (20 ml) is added to a mixture of 2-n-butyl-4-methoxynaphthol (1.0 g, 4.35 mmole), t-Boc-L-Valine (1.1 eq, 1.04 g), dicyclohexylcarbodiimide (1.1 eq, 0.985 g), prepared under an argon atmosphere, at 0° C (ice bath). The ice bath is removed and the resulting solution is stirred at room temperature for 18 hours und r argon. The white precipitate is removed by filtration through the celite, which is washed with 200 ml of ether. The filtrate is washed twice with 50 ml of brine, dried over anhydrous sodium sulfate, filtered and concentrated. The yellow residue is chromatographed through a silica gel flash column and eluted by 10% ether in n-hexane, to give 1.29 g (69.0%) of t-Boc-L-Valine, 2-n-butyl-4-methoxynaphth-1-yl ester as a pale yellow syrup.

Physical characteristics are as follows:
Mass spectra (m/e): 356, 286, 230, and 187.
IR ($cm^{-1}$): 1760, 1718, 1635, 1601, 1509, and 1462.
$^1$H-NMR ($\delta$, $CDCl_3$): 8.30-8.10, 7.75-7.35, 6.64, 5.00, 4.75-4.55, 3.98, 2.70-2.45, 1.48, 1.70-1.40, and 1.30-0.90.

Part B

Trifluoroacetic acid (25 ml) is added to a cooled (ice bath) solution of the t-Boc-L-Valine, 2-n-butyl-4-methoxynaphth-1-yl ester (1.24 g, 2.89 mmole) in dry methylene chloride (50 ml), prepared under an argon atmosphere. The ice bath is removed, and the resulting solution is stirred at room temperature for 1 hour under argon. The solvents are removed using a rotary evaporator, and the residue is dissolved in 150 ml of methylene chloride. The methylene chloride extracts are washed with 75 ml of water, saturated aqueous sodium bicarbonate solution (75 ml), then twice with 75 ml of water, dried over anhydrous sodium sulfate, filtered and concentrated. The residue is chromatographed through a silica gel flash column and eluted by 0.3% methanol in methylene chloride to give 0.94 g (99.0%) of L-Valine, 2-n-butyl-4-methoxynaphth-1-yl ester as a white solid.

Physical characteristics are as follows:
Mass spectra (m/e): 329, 230, 187, 173, and 72.
IR ($cm^{-1}$): 3411, 1751, 1466, 1379, 1224, and 1137.
$^1$H-NMR ($\delta$, $CDCl_3$): 8.35-8.10, 7.75-7.30, 6.65, 3.99, 3.76, 2.70-2.35, 1.70-1.40, and 1.35-0.90.

The free amine is treated with hydrochloric acid gas in anhydrous ether, and the resulting hydrochloric acid salt is recrystallized from methylene chloride and n-hexane in accordance with the procedure of Example 13, Part B.

Physical characteristics are as follows:
Melting point: 205-209° C.
Mass spectra (m/e): 329, 230, 215, 187, and 72.
IR ($cm^{-1}$): 1756, 1635, 1463, 1377, and 1213.

Anal. Calc'd. for $C_{20}H_{28}NO_3Cl$: C, 65.65; H, 7.71; N, 3.83; Cl, 9.69.
Found : C, 66.01; H, 7.99; N, 3.68; Cl, 9.45.

Example 17 L-Alanine, 2-n-butyl-4-methoxynaphth-1-yl ester and its hydrochloric acid salt (Formula I: D is -CH=CH-, $R_3$ is $-C(O)-(CR_{17}R_{18})_m-(CH_2)_n-NR_{14}R_{15}$, $R_2$ is butyl, $R_1$ is hydrogen, $R_{17}$ is hydrogen, $R_{18}$ is methyl, $R_{14}$ is hydrogen, $R_{15}$ is hydrogen, n is 0, and m is 1).

Part A

Dry methylene chloride (30 ml) is added to a mixture of 2-n-butyl-4-methoxynaphthol (1.697 g, 7.37 mmole), t-Boc-L-Alanine (1.1 eq, 1.56 g), dicyclohexylcarbodiimide (1.1 eq, 1.68 g) and 4-dimethylaminpyridine (0.1 eq, 93 mg) under an argon atmosphere at 0° C (ice bath). The ice bath is removed, and the resulting solution is stirred at room temperature for 18 hours under argon. The white precipitate is removed through the celite, which is washed with 200 ml of methylene chloride. The combined filtrates are washed three times with water, dried over magnesium sulfate, filtered and concentrated. The residue is chromatographed through a silica gel flash column and eluted by 20-35% ether in n-hexane, to give 2.51 g (84.8%) of t-Boc-L-Alanine, 2-n-butyl-4-methoxynaphth-1-yl ester as a colorless syrup.
Physical characteristics are as follows:
Mass spectra (m/e): 230, 187, and 57.
IR ($cm^{-1}$): 3474, 1751, 1679, 1518, 1375, and 1298.
$^1$H-NMR (δ, $CDCl_3$): 8.25-8.15, 7.75-7.30, 6.64, 5.20-5.00, 4.85-4.55, 3.99, 2.75-2.40, 1.70, 1.48, and 1.10-0.85.

Anal. Calc'd. for $C_{23}H_{31}NO_5$: C, 68.80; H, 7.78; N, 3.47.
Found : C, 68.95; H, 7.97; N, 3.42.

Part B

Trifluoroacetic acid (25 ml) is introduced under an argon atmosphere to a cooled (ice bath) solution of the t-Boc-L-Alanine,2-n-butyl-4-methoxynaphth-1-yl ester (2.5 g, 6.23 mmole) in dry methylene chloride (25 ml). The ice bath is removed and the resulting solution is stirred at room temperature for 1 hour under argon, and the solvents are removed using a rotary evaporator and the residue is dissolved in methylene chloride. The methylene chloride extracts are washed once with water, once with saturated aqueous sodium bicarbonate solution, and twice with water, dried over anhydrous sodium sulfate, filtered and concentrated. The residue is chromatographed through a silica gel (225 g) flash column and eluted by 30% ethyl acetate in methylene chloride, to give 1.64 g (87.3%) of L-Alanine, 2-n-butyl-4-methoxynaphth-1-yl ester as a colorless oil.
Physical characteristics are as follows:
Mass spectra (m/e): 301, 230, and 187.
IR ($cm^{-1}$): 3380, 1756, 1601, 1461, 1376, 1221, and 1161.
$^1$H-NMR (δ, $CDCl_3$): 8.35-8.10, 7.75-7.30, 6.64, 3.99, 4.15-3.90, 2.75-2.45, 1.67, 1.90-1.20, and 1.10-0.80.

Anal. Calc'd. for $C_{18}H_{23}NO_3$: 301.1678.
Found : 301.1682.

In accordance with the procedure of Example 13, Part B, the alanine ester is converted to the hydrochloric acid salt, and recrystallized from methanol and ether.

Physical characteristics are as follows:
Melting point: 185-187° C.
Mass spectra (m/e): 301, 230, 215, and 187.
IR ($cm^{-1}$): 1765, 1638, 1504, 1460, 1376, and 1188.

Anal. Calc'd. for $C_{18}H_{24}NClO_3$: C, 63.99; H, 7.16; N, 4.15; Cl, 10.49.
Found : C, 63.89; H, 7.38; N, 4.05; Cl, 9.85.

Example 18 L-Phenylalanine, 2-n-butyl-4-methoxynaphth-1-yl ester and its hydrochloric acid salt (Formula I: D is -CH=CH-, $R_3$ is $-C(O)-(CR_{17}R_{18})_m-(CH_2)_n-NR_{14}R_{15}$, $R_2$ is butyl, $R_1$ is hydrogen, $R_{17}$ is hydrogen, $R_{18}$ is benzyl, $R_{14}$ is hydrogen, $R_{15}$ is hydrogen, n is 0, and m is 1).

Part A

Dry methylene chloride (30 ml) is added to a mixture of 2-n-butyl-4-methoxynaphthol (1.0 g, 4.3 mmole), t-Boc-L-phenylalanine (1.5 eq, 1.7 g, 6.4 mmole), dicyclohexylcarbodiimide (1.5 eq, 1.4 g, 6.4 mmole) and 4-dimethylaminpyridine (0.74 eq, 0.04 g, 3.2 mmole), under an argon atmosphere at room temperature. The resulting solution is stirred at room temperature for 20 hours under argon. After the white precipitates are removed by filtration, the filtrate is washed twice with 100 ml of saturated aqueous sodium bicarbonate solution, three times with 100 ml of water, dried over anhydrous sodium sulfate, filtered and concentrated. The residue is chromatographed through a silica gel (200 g) flash column and eluted by 10-15% ether in n-hexane to give 1.78 g (86.8%) of t-Boc-L-phenylalanine, 2-n-butyl-4-methoxynaphth-1-yl ester as a white amorphous crystal.

Physical characteristics are as follows:
Mass spectra (m/e): 404, 230, and 187.
IR ($cm^{-1}$): 3344, 1770, 1695, 1535, 1463, and 1156.
$^1$H-NMR (δ, $CDCl_3$): 8.25-8.00, 7.50-7.20, 6.63, 5.20-4.90, 3.98, 3.60-3.20, 2.60-2.40, 1.43, 1.60-1.30, and 1.05-0.85.

Anal. Calc'd. for $C_{29}H_{35}NO_5$: C, 72.93; H, 7.39; N, 2.93.

Found : C, 72.88; H, 7.65; N, 2.83.

Part B

The cooled (ice bath) solution of the t-Boc-L-Phenylanine,2-n-butyl-4-methoxynaphth-1-yl ester (1.68 g, 3.5 mmole) in dry methylene chloride (40 ml), prepared under an argon atmosphere, is treated with trifluoroacetic acid (10 ml). The resulting yellow solution is stirred at 0° C for 30 minutes under argon. The ice bath is removed, and the reaction solution is stirred at room temperature for an additional 45 minutes under argon. The solvents are removed using a rotary evaporator, and the residue is dissolved in 300 ml of

methylene chloride. The methylene chloride extracts are washed three times with 200 ml of water, dried over anhydrous sodium sulfate, filtered and concentrated. The residue is chromatographed through a silica gel (200 g) flash column and eluted by 1% methanol in methylene chloride, to give 1.3 g (98.5%) of L-phenylalanine, 2-n-butyl-4-methoxynaphth-1-yl ester as a pale yellow oil.

Physical characteristics are as follows:
Mass spectra (m/e): 230, 214, 120, and 91.
IR ($cm^{-1}$): 3380, 3320, 3060, 3030, 3000, 2960, 2940, 2860, 1755, 1635, 2600, 1585, 1510, 1495, 1460, 1375, 1220, 700, 750, and 700.
$^1$H-NMR ($\delta$, $CDCl_3$): 8.30-8.15, 7.65-7.25, 6.64, 4.30-4.00, 3.99, 3.60-2.90, 2.65-2.40, 1.70-1.20, and 1.00-0.75.

In accordance with the procedure of Example 13, Part B, the free amine is converted to the hydrochloric acid salt, and recrystallized from absolute ethanol and ether in n-hexane.

Physical characteristics are as follows:
Melting point: 225-227° C.
Mass spectra (m/e): 377, 349, 230, 187, and 120.
IR ($cm^{-1}$): 3060, 2800, 2720, 2660, 2650, 2060, 1760, 1635, 1600, 1525, 1220, 1190, 1165, 1120, 770, 755, 730, and 700.

Anal. Calc'd. for $C_{24}H_{28}ClNO_3$: C, 69.64; H, 6.82; N, 3.38; Cl, 8.57.
Found : C, 69.39; H, 6.91; N, 3.46; Cl, 8.64.

Example 19 Following the procedures of the preceding Examples, and those depicted in Chart A, all of the compounds of this invention are prepared.

Example 20 Treatment of deep vein thrombosis using 2,3-diethyl-4-methoxy-1-naphthalenol, acetate

Nine adult domestic short hair cats weighing 4.5-5 kg were anesthetized with sodium pentabarbital (25-30 mg/kg i.v.). The neck area was prepared for a sterile cut down procedure and the jugular veins were exposed. After exposure of the veins each animal was injected with epsilon amino caproic acid (eaca) to inhibit plasminogen activation (2.5 grams i.v. in Tyrode's solution). This was done to reduce the normally highly active feline fibrinolytic mechanism and, thus reduce lysis of fibrin thrombi which might form in the vessel. Six of the cats were also treated with 2,3-diethyl-4-methoxy-1-naphthalenol, acetate. Three of the cats were given 1 mg/kg i.v. and 3 of the cats were treated with 5 mg/kg i.v. prior to venous occlusion. The jugular veins were carefully dissected free of surrounding connective tissue and the vein was ligated at the thoracic inlet with 3-0 dexon. The skin incision was closed and the animals were maintained in surgical anesthesia for 2 hours. At the end of the 2 hour waiting period, the jugular veins were exposed again. A 25 gauge butterfly needle was positioned into the lumen and blood was flushed out of the vein with heparinized Tyrode's solution (1U heparin/ml). The veins were tied off and removed under physiologic pressure. The vessels were immediately immersed in a 2.5% glutaraldehyde solution prepared in Tyrode's solution for fixation. The veins were fixed overnight at 5 degrees centigrade, postfixed in 1% osmium tetroxide overnight, dehydrated in ethanol and substituted by amyl acetate. All veins were critical point dried from carbon dioxide using a Denton DCP-1 critical point drying apparatus. The veins were mounted on stubas, gold coated on a Denton DV-502 modified vacuum evaporator and examined on a Cambridge Stereoscan 150 scanning electron microscope.

Two hours of jugular vein stasis following the trauma of dissection caused massive white cell adhesion to and migration under the venous endothelium of non-treated cats and produced sloughing of the endothelium and exposure of the basement membrane. This damage was sufficient to initiate thrombosis. Many of the exposed areas of basement membrane had adherent platelets. The platelets were adherent as monolayers or as aggregates. In some areas fibrin was found deposited on the endothelium or exposed basement membrane. Leukocytes were occasionally associated with the fibrin.

2,3-Diethyl-4-methoxy-1-naphthylenol, acetate had a dose dependent effect on the vascular changes described above. The 1 mg/kg dose did not reduce leukocyte adhesion or migration. Most of the surface of all the veins examined in this group had multiple aggregates of leukocytes visible under the intact

endothelium. Although leukocyte migration was not inhibited, leukocyte release of hydrolytic enzymes and production of superoxide's was apparently inhibited. Few areas with detached endothelial cells or exposed basement membrane were found in this group.

The 5 mg/kg dose had an even greater inhibitory effect on leukocyte mediated damage. In this group only patchy areas of adherent leukocytes were observed. In most of these areas leukocytes were also found under the endothelium. It was only in these areas, however, that endothelial cell loss and platelet deposition were observed. The numbers of adhering and migrating leukocytes found in this group appeared to be less than in either the control or 1 mg/kg treated group. With the exception of one vein, approximately half or more of the vascular surface had an intract endothelial cell cover with few adherent leukocytes. Only occasional small areas of fibrin deposition were found in this group.

CHART A

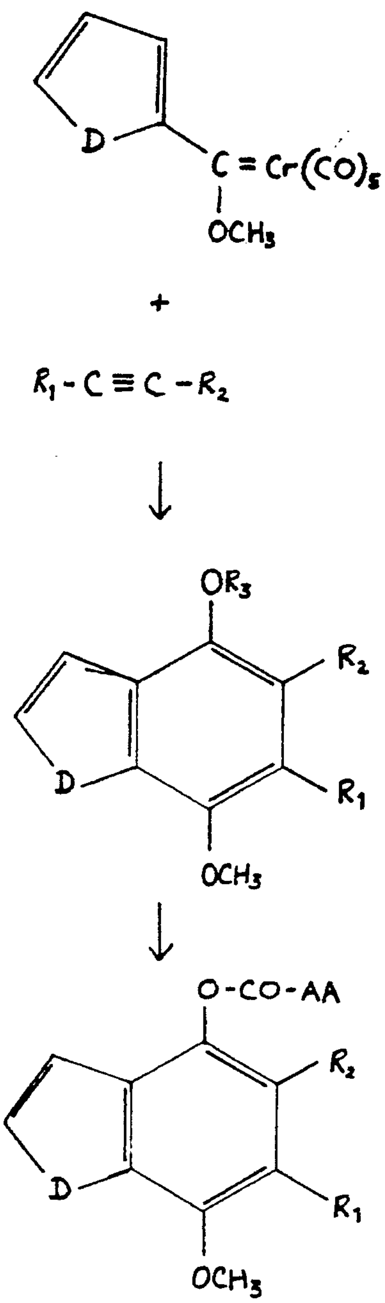

A-1

A-2

I (A-3)

Ia

## Claims

**1.** A compound, for therapeutic use, of the formula

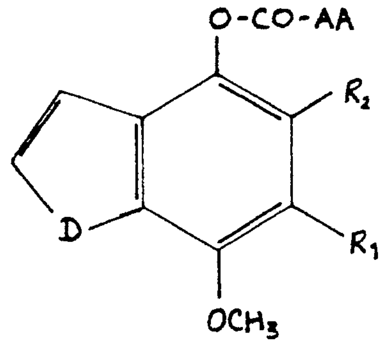

Ia

wherein $R_1$ and $R_2$ are independently selected from H, $C_{1-10}$ alkyl, $C_{2-10}$ alkenyl and PhX which represents phenyl optionally substituted by up to 3 substitutents independently selected from $C_{1-4}$ alkyl, Cl, F, Br, $NO_2$, $CF_3$, OH and $C_{1-4}$ alkoxy;

D is -CH=CH-, -N($CH_3$)-, -S- or -O-; and

AA is part of an amino-acid of the formulae HOOC-AA;

with the proviso that $R_1$ and $R_2$ are not both phenyl when D is -N($CH_3$)-;

or a pharmacologically acceptable acid addition salt thereof.

2. A compound, for therapeutic use, of the formula

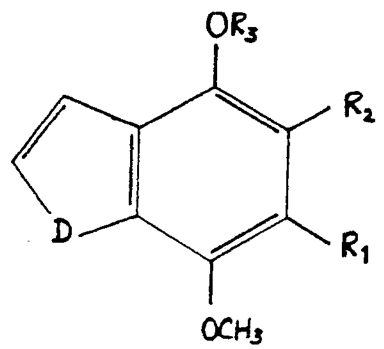

I

wherein $R_1$, $R_2$ and D are as defined in claim 1; and

$R_3$ is H or $OCCH_3$;

with the proviso that $R_3$ is not H when D is -N($CH_3$)-, and that $R_1$ and $R_2$ are not both phenyl when D is -N($CH_3$)-.

3. A compound of formula I as defined in claim 2, independent of use, with the additional provisos that $R_3$is not H when D is -CH=CH-, that $R_1$ is neither H nor $CH_3$ when $R_2$ is H or $CH_3$ and D is -CH=CH-, that $R_1$ is not H when $R_2$ is phenyl and D is -CH=CH-, and that $R_1$, $R_2$ and $R_3$ are not each H when D is -O-.

4. A compound of formula Ia as defined in claim 1, independent of use, with the additional proviso that $R_1$ is not H or alkyl when $R_2$ is H and D is -CH=CH-.

5. A compound of claim 1, wherein D is -CH=CH- and AA is $-(CR_{17}R_{18})_m-(CH_2)_n-NR_{14}R_{15}$, PhX-$NH_2$ or a naturally-occurring amino-acid minus its carboxylic acid function, in which m is 1, 2, 3 or 4, n is 0, 1, 2, 3, 4 or 5, $R_{14}$ and $R_{15}$ are independently selected from H, $C_{1-10}$ alkyl, ($C_{1-4}$ alkyl)carbonyl, -CO-Phx and -PhX, the or each $R_{17}$ and the or each $R_{18}$ are independently selected from H, $C_{1-10}$ alkyl, -$CH_2$-PhX and -PhX, and PhX-$NH_2$ represents PhX ring-substituted by $NH_2$, PhX being as defined in claim 1.

6. A compound of claim 2 or claim 3, wherein D is -CH=CH-.

7. A compound of any preceding claim, wherein $R_1$ and $R_2$ are each ethyl or $R_1$ is H and $R_2$ is n-butyl.

8. 4-Methoxy-2,3-diphenyl-1-naphthalenol, acetate, or 7-methoxy-1-methyl-5,6-diphenyl-1H-indol-4-ol, acetate (ester).

**9.** A compound selected from
5-butyl-7-methoxy-4-benzofuranol and its acetate,
5-butyl-7-methoxybenzo(b)thiophen-4-ol and its acetate,
5-butyl-7-methoxy-1-methyl-1H-indol-4-ol, acetate (ester), and 5,6-diethyl-7-methoxy-1-methyl-1H-indol-4-ol, acetate (ester).

**10.** 2-Butyl-4-methoxy-1-naphthalenol, acetate, or 2,3-diethyl-4-methoxy-1-naphthalenol, acetate.

**11.** A compound selected from
L-valine, 2,3-diethyl-4-methoxynaphth-1-yl ester and its hydrochloride,
L-alanine, 2,3-diethyl-4-methoxynaphth-1-yl ester,
L-phenylalanine, 2,3-diethyl-4-methoxynaphth-1-yl ester, hydrochloride,
3-(N,N-diethylamino)propionic acid, 2,3-diethyl-4-methoxynaphth-1-yl ester,
N,N-dimethylaminoacetic acid, 2,3-diethyl-4-methoxynaphth-1-yl ester,
L-valine, 2-n-butyl-4-methoxynaphth-1-yl ester and its hydrochloride,
L-alanine, 2-n-butyl-4-methoxynaphth-1-yl ester and its hydrochloride, and
L-phenylalanine, 2-n-butyl-4-methoxynaphth-1-yl ester, hydrochloride.

**12.** L-valine, 2,3-diethyl-4-methoxynaphth-1-yl ester or its hydrochloride.

**13.** L-alanine, 2-n-butyl-4-methoxynaphth-1-yl ester or its hydrochloride.

## Revendications

**1.** Composé à usage thérapeutique de formule

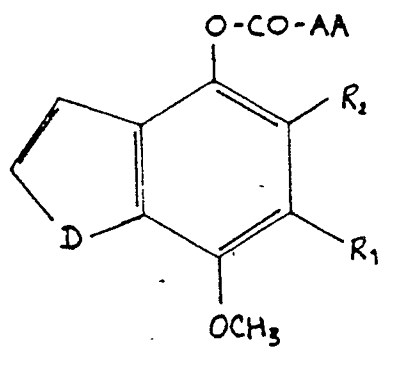

Ia

dans laquelle $R_1$ et $R_2$ sont choisis indépendamment entre H, des groupes alkyle en $C_1$ à $C_{10}$, alcényle en $C_2$ à $C_{10}$ et PhX qui représente le groupe phényle portant facultativement jusqu'à trois substituants choisis indépendamment entre des substituants alkyle en $C_1$ à $C_4$, Cl, F, Br, $NO_2$, $CF_3$, OH et alkoxy en $C_1$ à $C_4$.

D représente -CH=CH-, -N($CH_3$)-, -S- ou -O- ; et

AA est une partie d'un amino-acide de formule HOOC-AA ;

sous réserve que $R_1$ et $R_2$ ne soient pas tous deux des groupes phényle lorsque D représente -N-($CH_3$)- ;

ou un sel d'addition d'acide pharmacologiquement acceptable.

**2.** Composé à usage thérapeutique de formule

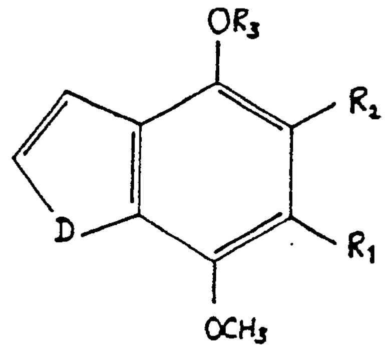

I

dans laquelle $R_1$, $R_2$ et D sont tels que définis dans la revendication 1 ; et

$R_3$ représente H ou $OCCH_3$ ;

sous réserve que $R_3$ ne soit pas H lorsque D est un groupe $-N(CH_3)-$ et que $R_1$ et $R_2$ ne soient pas tous deux des groupes phényle lorsque D est un groupe $-N(CH_3)-$.

**3.** Composé de formule I suivant la revendication 2, indépendamment de son usage, sous réserve en outre que $R_3$ ne représente pas H lorsque D représente -CH=CH-, que $R_1$ ne soit ni H ni $CH_3$ lorsque $R_2$ représente H ou $CH_3$ et D est un groupe -CH=CH-, que $R_1$ ne soit pas H lorsque $R_2$ est un groupe phényle et D est un groupe -CH=CH-, et que $R_1$, $R_2$ et $R_3$ ne représentent pas chacun H lorsque D représente -O-.

**4.** Composé de formule Ia suivant la revendication 1, indépendamment de son usage, sous réserve en outre que $R_1$ ne représente pas H ou un groupe alkyle lorsque $R_2$ est H et D est un groupe -CH=CH-.

**5.** Composé suivant la revendication 1, dans lequel D est un groupe -CH=CH- et AA est un groupe $-(CR_{17}R_{18})_m-(CH_2)_n-NR_{14}R_{15}$, $PhX-NH_2$ ou un amino-acide naturel moins sa fonction acide carboxylique, m a la valeur 1, 2, 3 ou 4 et n'a la valeur 0, 1, 2, 3, 4 ou 5, $R_{14}$ et $R_{15}$ sont choisis indépendamment entre H, des groupes alkyle en $C_1$ à $C_{10}$, (alkyle en $C_1$ à $C_4$)carbonyle, -CO-PhX et -PhX, le groupe $R_{17}$ ou chaque groupe $R_{17}$ et le groupe $R_{18}$ ou chaque groupe $R_{18}$ est choisi indépendamment entre H, des groupes alkyle en $C_1$ à $C_{10}$, $-CH_2-PhX$ et -PhX, et $PhX-NH_2$ représente le noyau PhX substitué par un radical $NH_2$, PhX étant tel que défini dans la revendication 1.

**6.** Composé suivant la revendication 2 ou la revendication 3, dans lequel D est un groupe -CH=CH-.

**7.** Composé suivant l'une quelconque des revendications précédentes, dans lequel $R_1$ et $R_2$ représentent chacun un groupe éthyle ou bien $R_1$ représente H et $R_2$ est un groupe n-butyle.

**8.** L'acétate de 4-méthoxy-2,3-diphényl-1-naphtalénol ou l'acétate (ester) de 7-méthoxy-1-méthyl-5, 6-diphényl-1H-indole-4-ol.

**9.** Composé choisi entre

le 5-butyl-7-méthoxy-4-benzofurannol et son acétate, le 5-butyl-7-méthoxybenzo(b)thiophène-4-ol et son acétate, l'acétate (ester) de 5-butyl-7-méthoxy-1-méthyl-1H-indole-4-ol et l'acétate (ester) de 5,6-diéthyl-7-méthoxy-1-méthyl-1H-indole-4-ol.

**10.** L'acétate de 2-butyl-4-méthoxy-1-naphtalénol ou l'acétate de 2,3-diéthyl-4-méthoxy-1-naphtalénol.

**11.** Composé choisi entre

l'ester de 2,3-diéthyl-4-méthoxynapht-1-yle de L-valine et son chlorhydrate, l'ester de 2,3-diéthyl-4-méthoxynapht-1-yle de L-alanine, le chlorhydrate de l'ester de 2,3-diéthyl-4-méthoxynapht-1-yle de L-phénylalanine, l'ester de 2,3-diéthyl-4-méthoxynapht-1-yle de l'acide 3-(N,N-diéthylamino)propionique, l'ester de 2,3-diéthyl-4-méthoxynapht-1-yle de l'acide N,N-diméthylaminoacétique, l'ester de 2-n-butyl-4-méthoxynapht-1-yle de L-valine et son chlorhydrate, l'ester de 2-n-butyl-4-méthoxynapht-1-yle de L-alanine et son chlorhydrate, et le chlorhydrate d'ester de 2-n-butyl-4-méthoxynapht-1-yle de L-phénylalanine.

**12.** L'ester de 2,3-diéthyl-4-méthoxynapht-1-yle de L-valine ou son chlorhydrate.

**13.** L'ester de 2-n-butyl-4-méthoxynapht-1-yle de L-alanine ou son chlorhydrate.

## Patentansprüche

**1.** Verbindung, für therapeutischen Gebrauch, mit der Formel

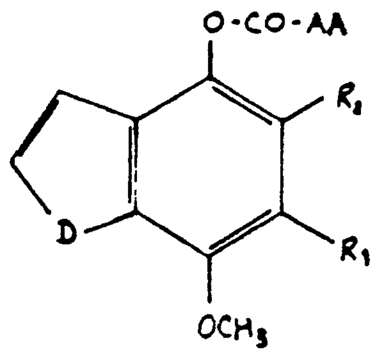

Ia

worin $R_1$ und $R_2$ unabhängig von H, $C_{1-10}$ Alkyl, $C_{2-10}$Alkenyl und PhX ausgewählt werden, wobei PhX Phenyl darstellt, wahlweise durch bis zu 3 Substituenten ersetzt, die unabhängig von $C_{1-4}$Alkyl, Cl, F, Br, $NO_2$, $CF_3$, OH und $C_{1-4}$Alkoxy ausgewählt werden,
D -CH=CH-, -N($CH_3$)-, -S- oder -O- ist; und
AA Teil einer Aminosäure der Formel HOOC-AA ist;
unter der Bedingung, dass $R_1$ und $R_2$ nicht beide Phenyl sind, wenn D -N($CH_3$)- ist;
oder ein pharmakologisch akzeptables Säurenzusatzsalz davon.

**2.** Verbindung, für therapeutischen Gebrauch, der Formel

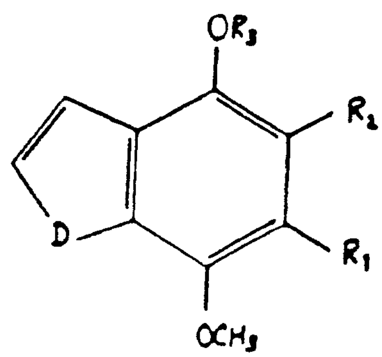

I

worin $R_1$, $R_2$ und D wie in Anspruch 1 definiert sind; und
$R_3$ H oder $OCCH_3$ ist;
unter der Bedingung, dass $R_3$ nicht H ist, wenn D -N($CH_3$)-ist, und dass $R_1$ und $R_2$ nicht beide Phenyl sind, wenn D -N($CH_3$)- ist.

**3.** Verbindung der Formel I, wie definiert in Anspruch 2, von Gebrauch unabhängig , unter den zusätzlichen Bedingungen, dass $R_3$ nicht H ist, wenn D -CH=CH- ist, dass $R_1$ weder H noch $CH_3$ ist, wenn $R_2$ H oder $CH_3$ ist und D -CH=CH- ist, dass $R_1$ nicht H ist, wenn $R_2$ Phenyl ist und D -CH=CH- ist, und dass $R_1$, $R_2$ und $R_3$ nicht je H sind, wenn D -O- ist.

**4.** Verbindung der Formel Ia wie definiert in Anspruch 1, von Gebrauch unabhängig , unter der zusätzlichen Bedingung, dass $R_1$ nicht H oder Alkyl ist, wenn $R_2$ H ist und D -CH=CH- ist.

**5.** Verbindung nach Anspruch 1, worin D -CH=CH- ist und AA -($CR_{17}R_{18}$)$_m$-($CH_2$)$_n$-$NR_{14}R_{15}$, PhX-$NH_2$

oder eine natürlich vorkommende Aminosäure minus ihrer Carbonsäurenfunktion ist, in welcher m 1, 2, 3, oder 4 ist, n 0, 1, 2, 3, 4 oder 5 ist, $R_{14}$ und $R_{15}$ unabhängig von H, $C_{1-10}$Alkyl, ($C_{1-4}$Alkyl)-Carbonyl, -CO-PhX und -PhX ausgewählt werden, das oder jedes $R_{17}$ und das oder jedes $R_{18}$ unabhängig von H, $C_{1-10}$Alkyl, -$CH_2$-PhX und PhX ausgewählt werden, und PhX-$NH_2$ PhX ring-substituiert durch $NH_2$ darstellt, wobei PhX wie in Anspruch 1 definiert ist.

6. Verbindung nach Anspruch 1 oder 2, worin D -CH = CH- ist.

7. Verbindung nach einem der vorhergehenden Ansprüche, worin $R_1$ und $R_2$ je Ethyl sind, oder $R_1$ H ist und $R_2$ n-Butyl ist.

8. 4-Methoxy-2,3-Diphenyl-1-Naphthalenol, Acetat, oder 7-Methoxy-1-Methyl-5,6-Diphenyl-1H-Indol-4-ol, Acetat (Ester).

9. Verbindung, ausgewählt von
5-Butyl-7-Methoxy-4-Benzofuranol und sein Acetat,
5-Butyl-7-Methoxybenzo(b)thiopen-4-ol und sein Acetat,
5-Butyl-7-Methoxy-1-Methyl-1H-Indol-4-ol, Acetat (Ester), und
5,6-Diethyl-7-Methoxy-1-Methyl-1H-Indol-4-ol, Actetat (Ester).

10. 2-Butyl-4-Methoxy-1-Naphthalenol, Acetat oder 2,3-Diethyl-4-Methoxy-1-Naphthalenol, Acetat.

11. Verbindung, ausgewählt von
L-Valin, 2,3-Diethyl-4-Methoxynaphth-1-yl Ester und seinem Hydrochlorid,
L-Alanin, 2,3-Diethyl-4-Methoxynaphth-1-yl Ester,
L-Phenylalanin, 2,3-Diethyl-4-Methoxynaphth-1-yl Ester, Hydrochlorid,
3-(N,N-Diethylamino)propionische Säure, 2,3-Diethyl-4-Methoxynaphth-1-yl Ester,
N,N-Dimethylaminoacetische Säure, 2,3-Diethyl-4-Methoxynaphth-1-yl Ester,
L-Valin, 2-n-Butyl-4-Methoxynaphth-1-yl Ester und sein Hydrochlorid,
L-Alanin, 2-n-Butyl-4-Methoxynaphth-1-yl Ester und sein Hydrochlorid, und
L-Phenylalanin, 2-n-Butyl-4-Methoxynaphth-1-yl Ester, Hydrochlorid.

12. L-Valin, 2,3-Diethyl-4-Methoxynaphth-1-yl Ester oder sein Hydrochlorid.

13. L-Alanin, 2-n-Butyl-4-Methoxynaphth-1-yl Ester oder sein Hydrochlorid.